# FASCICULE DE BREVET EUROPEEN

(11) **EP 3 600 336 B1**
(45) Date de publication et mention de la délivrance du brevet: **01.01.2025**
(21) Numéro de dépôt: 18722117.1
(22) Date de dépôt: 30.03.2018
(51) Int. Cl.: A61K 31/7016, A61K 31/702, A61P 3/08, A61P 1/18

(54) **ADMINISTRATION PÉRINATALE DE FRUCTOOLIGOSACCHARIDES À CHAÎNE COURTE AFIN D'EMPÊCHER DES TROUBLES MÉTABOLIQUES LIÉS À UN RÉGIME ALIMENTAIRE DÉSÉQUILIBRÉ À L'ÂGE ADULTE**
PERINATALE VERABREICHUNG VON KURZKETTIGEN FRUCTOOLIGOSACCHARIDEN ZUR VORBEUGUNG VON STOFFWECHSELSTÖRUNGEN IN VERBINDUNG MIT EINER UNAUSGEGLICHENEN ERNÄHRUNG IM ERWACHSENENALTER
PERINATAL ADMINISTRATION OF SHORT-CHAIN FRUCTOOLIGOSACCHARIDES IN ORDER TO PREVENT METABOLIC DISORDERS LINKED TO AN UNBALANCED DIET AT ADULT AGE

(30) Priorité: 31.03.2017 FR 1700354
(43) Date de publication de la demande: 05.02.2020
(73) Titulaire: Beghin Meiji, 77230 Moussy-le-Vieux (FR); Institut national de recherche pour l'agriculture, l'alimentation et l'environnement, 75007 Paris (FR)
(72) Inventeur: LE BOURGOT, Cindy, 77230 Moussy-le-Vieux (FR); APPER, Emmanuelle, 82220 Labarthe (FR); LE HUËROU-LURON, Isabelle, 35590 Saint-Gilles (FR); BLAT, Sophie, 35590 Saint-Gilles (BE)
(74) Mandataire: Icosa
(86) Numéro de dépôt international: PCT/FR2018/050805
(87) Numéro de publication internationale: WO 2018/178599

(56) Documents cités:
- WO-A1-2016/030861
- WO-A2-2007/050656
- US-A1- 2013 216 521
- DENNISON CAROL ET AL: "Fetal programming of metabolic health through a combined dietary and pharmacological pre-pregnancy intervention in rats", FASEB JOURNAL, vol. 27, April 2013 (2013-04-01), & JOINT ANNUAL MEETING OF THE ASPET/BPS AT EXPERIMENTAL BIOLOGY (EB); BOSTON, MA, USA; APRIL 20 -24, 2013, pages 111.6, XP009502456
- WERNIMONT SUSAN ET AL: "Effects of Term Infant Formula Supplemented with Oligofructose on Intestinal Microbiota Composition, Diversity, and Metabolic Activity", FASEB JOURNAL, vol. 30, no. Suppl. 1, April 2016 (2016-04-01), & EXPERIMENTAL BIOLOGY MEETING; SAN DIEGO, CA, USA; APRIL 02 -06, 2016, pages lb235, XP009502457
- MENNITTI L V: "Maternal supplementation with oligofructose (10%) during pregnancy and lactation leads to increased pro-inflammatory status of the 21-D-old offspring", PLOS ONE 20150706 PUBLIC LIBRARY OF SCIENCE USA, vol. 10, no. 7, 6 July 2015 (2015-07-06), XP055436133, ISSN: 1932-6203
- HALLAM MEGAN C ET AL: "Postnatal prebiotic fiber intake in offspring exposed to gestational protein restriction has sex-specific effects on insulin resistance and intestinal permeability in rats.", THE JOURNAL OF NUTRITION OCT 2014, vol. 144, no. 10, October 2014 (2014-10-01), pages 1556 - 1563, XP009502451, ISSN: 1541-6100

## Description

### DOMAINE DE L'INVENTION

L'invention concerne la prévention de troubles métaboliques liés à un régime alimentaire déséquilibré à l'âge adulte d'une progéniture par l'administration de ScFOS pendant la période périnatale.

### ÉTAT DE LA TECHNIQUE

Le concept des origines développementales de la santé et des maladies (DOHaD), sur la base d'indications épidémiologiques substantielles et de données expérimentales (Gluckman *et al.*, 2007 ; Godfrey *et al.*, 2010), stipule que la santé métabolique de l'adulte peut être programmée par des facteurs environnementaux, y compris la nutrition, lors de la vie périnatale. Les stades du début de la vie, à partir de la vie foetale jusqu'à la fin de la petite enfance chez l'homme (2 ans), constituent une fenêtre critique de sensibilité vis-à-vis d'une telle programmation.

Les données expérimentales pointent vers le pancréas endocrine comme étant un tissu important et sujet à une imprégnation nutritionnelle. Le développement du pancréas endocrine commence au cours de la vie foetale et continue après la naissance. Par conséquent, des déficiences nutritionnelles, telles qu'une faible disponibilité en protéines ou une réduction de l'apport calorique, lors de la vie périnatale ont produit des altérations pancréatiques persistantes chez la progéniture adulte, qui ont inclus l'hypoplasie et l'hypotrophie des ilots de Langerhans pancréatiques avec des conséquences fonctionnelles sur l'homéostasie du glucose (Garofano *et al.*, 1998 ; Bertin *et al.*, 1999 ; Garofano *et al.,* 1999). La communication entre l'intestin et le pancréas, qui est obligatoire afin de maintenir l'homéostasie du glucose, est intégrée dans le concept de l'axe entéro-insulaire. Parmi les hormones intestinales libérées en réponse à l'ingestion de glucose, celles qui sont capables de potentialiser la sécrétion d'insuline induite par le glucose sont appelées incrétines, telles que le peptide-1 semblable au glucagon (GLP-1) sécrété à partir des cellules L de l'intestin grêle distal et du côlon proximal. Une fois sécrété, la fixation du GLP-1 à son récepteur spécifique sur les cellules β pancréatiques stimule la sécrétion d'insuline. Elle stimule aussi la prolifération et la néogenèse des cellules β, et inhibe leur apoptose, augmentant ainsi la masse des cellules β (Baggio & Drucker, 2007). Le GLP-1 inhibe la sécrétion de glucagon (hormone hyperglycémiante), induit la satiété et ralentit le vidange gastrique. La sécrétion du GLP-1 diminue la néoglucogénèse hépatique ainsi que le dépôt des acides gras au sein du tissu adipeux viscéral, responsable de l'obésité.

Le stimulus principal pour la sécrétion du GLP-1 est le glucose, mais sa libération peut également être stimulée par d'autres nutriments. En effet, Tolhurst *et al.* (Tolhurst *et al.*, 2012) ont démontré que les acides gras à chaîne courte (SCFA), et en particulier l'acétate et le propionate, qui peuvent être produits par une fermentation bactérienne dans les intestins, améliorent la libération du GLP-1 par les cellules L via l'activation du récepteur 2 des acides gras libres couplé à la protéine G. La fonction endocrine intestinale n'a pas été étudiée de manière approfondie dans le contexte de la programmation nutritionnelle par rapport à celle du pancréas. Cependant, des indications croissantes suggèrent que l'intestin est soumis à l'imprégnation nutritionnelle. Une déficience ou un excès nutritionnel précoce induit des conséquences à long terme sur la fonction intestinale, caractérisées par une barrière intestinale immunitaire et non immunitaire altérée (Fanca-Berthon *et al.*, 2009 ; Van *et al.*, 2011 ; Boudry *et al.*, 2013). La fonction entéro-endocrine pourrait donc constituer également un acteur important de l'imprégnation nutritionnelle, à cause de sa réponse adaptative aux nutriments du régime alimentaire et aux métabolites bactériens.

Le rôle du microbiote intestinal dans la programmation de la physiologie et du métabolisme est apparu récemment. L'environnement précoce tel que le mode d'administration, les habitudes alimentaires maternelles et les traitements antibiotiques périnataux affectent en effet la colonisation du microbiote des intestins dans des modèles chez l'homme et l'animal (Penders *et al.*, 2006 ; Reinhardt *et al.*, 2009 ; De Filippo *et al.,* 2010 ; Roura *et al.*, 2016). Une exposition à des antibiotiques lors de la petite enfance, par exemple, peut avoir des conséquences à long terme sur la composition du microbiote intestinal (Bedford Russell & Murch, 2006 ; Schokker *et al.*, 2015) et sur l'état de surpoids et métabolique (Ajslev *et al.*, 2011 ; Trasande *et al.*, 2013 ; Cox *et al.*, 2014). Cependant, peu d'informations sont disponibles sur les impacts métaboliques à long terme de stratégies nutritionnelles conçues pour favoriser la colonisation d'un microbiote bénéfique lors de la petite enfance.

Les fibres prébiotiques constituent de bons candidats afin de moduler le microbiote de manière positive. Il s'agit d'ingrédients fermentés de manière sélective qui permettent des changements spécifiques du microbiote intestinal, tant au niveau composition que métabolisme, conférant des bénéfices sur le bien-être et la santé de l'hôte (Roberfroid *et al.*, 2010). L'impact de la consommation d'un régime alimentaire complémenté par des prébiotiques lors de la grossesse et de la lactation sur la santé de la progéniture n'a pas été étudié de manière approfondie mais mérite une certaine attention à cause de l'influence que les prébiotiques exercent sur la composition et l'activité du microbiote. Les fructooligosaccharides à chaîne courte (scFOS) constituent des fibres prébiotiques hautement intéressantes, obtenus à partir de saccharose et constitués de 2 à 4 unités de fructose liées à une molécule de glucose, qui sont fermentés de manière sélective par le microbiote intestinal favorisant la croissance de *Lactobacillus, Bifidobacterium* et *Clostridium coccoides* principalement, ainsi que la production des acides gras à chaîne courte (SCFA) (Swanson *et al.*, 2002 ; Pan *et al.,* 2009 ; Respondek *et al.,* 2013). Le degré élevé de similarité entre le microbiote intestinal de mères complémentées par des scFOS et celui de leur progéniture chez des souris, deux semaines après la naissance (Fujiwara *et al.*, 2008) suggère que l'amélioration du profil du microbiote intestinal maternel peut améliorer la colonisation précoce des intestins de la progéniture par un transfert bactérien *in utero,* à la naissance, et lors de l'allaitement. Nous avons précédemment démontré chez le porc qu'une complémentation maternelle en scFOS induisait une activité métabolique accrue du microbiote de la progéniture lors de la lactation et une production accrue en butyrate après sevrage (Le Bourgot *et al.*, en révision), par rapport à la progéniture de truies non complémentées. Nous avons également observé un effet de longue durée de la complémentation périnatale en scFOS sur la production de métabolites du microbiote plus de 2 mois après l'arrêt de la complémentation (Le Bourgot *et al.*, 2014), suggérant un effet persistant d'une complémentation précoce en scFOS sur la composition du microbiote.

Depuis les années 60, il y a eu une transition au niveau alimentaire caractérisée par une augmentation de la consommation d'aliments ayant une densité énergétique élevée et souvent riches en matières grasses, et contenant peu de glucides complexes et de fibres. Cette transition diététique, associée à un manque d'exercice, contribue à l'augmentation de la fréquence de l'obésité et de ses troubles métaboliques associés tels que le diabète et les maladies cardio-métaboliques. De par le monde, le nombre de cas d'obésité a doublé depuis 1980. En 2014, plus de 1,9 milliard d'adultes étaient en surpoids, dont plus de 600 millions étaient obèses, représentant 39% des adultes qui étaient en surpoids et 13% qui étaient obèses. De plus, la fréquence globale du diabète parmi les adultes de plus de 18 ans a augmenté de 4,7% en 1980 jusqu'à 8,5% en 2014. D'ici 2030, l'OMS s'attend à ce que le nombre de personnes en surpoids atteigne 3,3 milliards et que le diabète devienne la septième cause principale de décès au niveau mondial.

Un autre facteur de risque de développement de troubles métaboliques a été mis en exergue plus récemment par la Fédération Internationale du Diabète. Il s'agit de l'environnement familial, et notamment de l'alimentation lors de la vie périnatale, qui pourrait représenter une stratégie nutritionnelle intéressante afin d'empêcher l'occurrence de maladies métaboliques à l'âge adulte.

La demande internationale WO2016/030861 suggère que l'administration de scFOS à un animal femelle lors de l'allaitement ou à sa progéniture, induit des effets sur le métabolisme de la progéniture, plus précisément l'anabolisme. Les effets métaboliques selon WO2016/030861 comprennent la prise de poids.

Nous avons donc étudié si une complémentation périnatale en scFOS pouvait avoir un impact sur la physiologie intestinale chez le mammifère, l'axe entéro-insulaire et l'homéostasie du glucose chez la progéniture adulte afin de limiter le développement de troubles métaboliques induits par un régime alimentaire déséquilibré.

Contrairement, à la divulgation de WO2016/030861 cette régulation du métabolisme grâce à l'invention est un équilibre entre les effets anaboliques et cataboliques. De manière surprenante, l'administration de scFOS à un sujet femelle ou à sa progéniture selon la présente invention, conduit à une régulation du métabolisme, malgré l'alimentation de la progéniture en tant qu'adulte. Le document DENNISON CAROL ET AL: "Fetal programming of metabolic health through a combined dietary and pharmacological pre-pregnancy intervention in rats",FASEB JOURNAL, vol. 27, avril 2013 (2013-04), page 111.6, XP009502456,& JOINT ANNUAL MEETING OF THE ASPET/BPS AT EXPERIMENTAL BIOLOGY (EB); BOSTON, MA, USA; APRIL 20 -24, 2013 1 décrit comment l'obésité maternelle est liée à un poids réduit à la naissance, au diabète et à l'obésité chez le nourrisson et comment les oligofructoses FOS améliorent le contrôle du glucose à travers le GLP-1 (glucagone-like peptide 1).

### RÉSUMÉ

L'invention concerne donc des fructooligosaccharides (FOS) pour leur utilisation à un âge précoce d'un animal dans la prévention des troubles métaboliques liés à un régime alimentaire déséquilibré à l'âge adulte ; lesquels troubles métaboliques étant sélectionnés d'un groupe comprenant l'hyperglycémie post-prandiale, l'hyperglycémie à jeun, l'hyperlipidémie, la surcharge pondérale, la surcharge de graisse sous-cutanée, la surcharge de graisse viscérale, le catabolisme de nutriments ralenti, ou leur combinaison, lesdits fructooligosaccharides étant administrés audit animal d'âge précoce entre 2 et 9 semaines après sevrage.

Selon l'invention, les fos FOS sont administrés en une quantité efficace à ladite progéniture pendant au moins deux semaines après le sevrage.

De manière surprenante, suite à l'administration des FOS selon l'invention à un âge précoce, les effets métaboliques sont maintenus jusqu'à l'âge adulte.

Cet effet est médié par l'équilibration de l'axe entéro-insulaire, de préférence par l'augmentation des hormones dites incrétines ou des cellules sécrétrices des incrétines. Dans un mode de réalisation, l'incrétine est le GLP-1.

Selon un mode de réalisation, la quantité efficace de FOS pour l'animal femelle lors de la gestation et/ou l'allaitement de sa progéniture est de 1 à 20 g, de préférence de 4 à 18 g de FOS par jour.

Selon un mode de réalisation, la quantité efficace est efficace pour la progéniture est de 0.1 à 2 g, de préférence de 0.3 à 1 g de FOS par jour.

L'animal d'après la présente invention est un mammifère, un mammifère humain ou non humain.

D'après un mode de réalisation, les fructooligosaccharides sont des fructooligosaccharides à chaîne courte.

L'invention concerne également des fructooligosaccharides (FOS) pour leur utilisation dans une méthode de stimulation de la fonction endocrine intestinale et l'augmentation de la sensibilité du pancréas vis-à-vis du glucose à l'âge adulte d'une progéniture issue d'un animal femelle, lesdits FOS étant administrés en une quantité efficace à
ladite progéniture pendant au moins deux semaines après le sevrage ; ladite stimulation de la fonction endocrine intestinale ou la dite augmentation de la sensibilité du pancréas vis-à-vis du glucose conduisent à l prévention des troubles métaboliques liés à un déséquilibre à un âge adulte de ladite progéniture ;
lesquels troubles métaboliques étant sélectionnés d'un groupe comprenant ou consistant en l'hyperglycémie post-prandiale, l'hyperglycémie à jeun, l'hyperlipidémie, la surcharge pondérale, la surcharge de graisse sous-cutanée, la surcharge de graisse viscérale, le catabolisme de nutriments ralenti, ou leur combinaison.

Dans un autre aspect, l'invention concerne des fructooligosaccharides (FOS) pour leur utilisation dans la préparation d'une composition alimentaire ou nutraceutique ; ladite composition étant administrée à un âge précoce d'un animal dans une méthode de prévention des troubles métaboliques liés à un régime alimentaire déséquilibré à l'âge adulte ; lesquels troubles métaboliques étant sélectionnés d'un groupe comprenant ou consistant en l'hyperglycémie post-prandiale, l'hyperglycémie à jeun, l'hyperlipidémie, la surcharge pondérale, la surcharge de graisse sous-cutanée, la surcharge de graisse viscérale, le catabolisme de nutriments ralenti, ou leur combinaison.

Cette composition est administrée en une quantité efficace à ladite progéniture pendant au moins deux semaines après le sevrage.L'administration de cette composition équilibre l'axe entéro-insulaire, de préférence par l'augmentation des hormones dites incrétines ou des cellules sécrétrices des incrétines, notamment le GLP-1 Cette composition comprend de 0.1 à 20 g de FOS ou de 0.05 à 2 % w/w en masse par rapport à la composition.

La demande divulgue également mais ne revendique pas l'utilisation des FOS pour la préparation d'une composition telle que décrite précédemment.

### DESCRIPTION DÉTAILLÉE

En complémentant des truies par des scFOS lors du dernier tiers de la gestation et de l'ensemble de la lactation, et/ou en complémentant leur progéniture pendant un mois après sevrage, les inventeurs ont montré les effets d'une complémentation précoce en scFOS sur la programmation de la fonction entéro-endocrine et leurs conséquences métaboliques sur la santé de porcs adultes soumis à un régime alimentaire déséquilibré.

L'objet de l'invention est décrit dans les revendications uniquement. Toute référence à une méthode de traitement est à comprendre comme une référence à la composition ou au composé pour son utilisation dans ladite méthode de traitement.

L'invention concerne des fructooligosaccharides pour la prévention de troubles métaboliques liés à un régime alimentaire déséquilibré à l'âge adulte d'une progéniture issue d'un animal femelle, comprenant l'étape consistant à
- administrer une quantité efficace de fructooligosaccharides à ladite progéniture pendant entre deux et neuf semaines après le sevrage et optionnellement à l'animal femelle lors de la gestation et/ou l'allaitement de ladite progéniture;
- empêcher des troubles métaboliques liés à un déséquilibre à un âge adulte de ladite progéniture.

L'invention concerne également l'utilisation de fructooligosaccharides (FOS) préférablement de FOS à chaîne courte (ScFOS) à un âge précoce d'un animal afin d'empêcher des troubles métaboliques liés à un régime alimentaire déséquilibré à l'âge adulte. Préférablement par l'administration audit animal d'une quantité efficace de fructooligosaccharides pendant au moins un mois après sevrage. L'invention concerne également l'utilisation de FOS, préférablement de ScFOS selon les revendications, afin d'empêcher des troubles métaboliques liés à un régime alimentaire déséquilibré à l'âge adulte d'un animal, par l'administration de ScFOS à son géniteur femelle (ou mère, ou génitrice) lors de l'allaitement dudit animal.

Selon l'invention, les fructooligosaccharides sont administrés à ladite progéniture ou audit animal d'âge précoce entre 2 et 9 semaines après sevrage, préférablement entre 3 et 8 semaines après sevrage, encore plus préférablement de 4 à 7 semaines.

Selon l'invention, les fructooligosaccharides sont administrés audit animal femelle ou à ladite mère au moins lors de la période de lactation, préférablement les fructooligosaccharides sont administrés audit animal femelle ou à ladite mère lors de la fin de la gestation et/ou lors de l'ensemble de la période de lactation. Dans un mode de réalisation la mère n'est pas obèse, et présente un statut métabolique normal

Tel qu'utilisé ici, par le terme « **animaux** » on entend l'homme ou tout animal élevé dans la technique de l'élevage animal pour une utilisation par l'homme pour l'alimentation, l'habillement, et similaires ; de tels animaux comportent ceux producteurs de viande, par exemple, les ruminants tels que les bovins, les ovins et les caprins et les non ruminants tels que les porcs et la volaille. Préférablement, l'animal est un animal non humain. Préférablement l'animal est un mammifère. Par le terme « animaux producteurs de viande » on entend tout animal élevé dans la technique de l'élevage animal pour une utilisation de sa viande comme aliment. Typiquement, l'animal est un animal monogastrique. Par le terme « animal monogastrique » on entend tout animal ayant un seul estomac, ce qui s'applique à la plupart des carnivores et omnivores, à l'exception des ruminants sauf les veaux de boucherie, en fait le terme « animal monogastrique » doit être considéré comme incluant les veaux de boucherie.

Par le terme « **régime alimentaire déséquilibré** » on entend un régime alimentaire composé d'une proportion élevée d'énergie dérivée de lipides et/ou de glucides. Préférablement, le régime alimentaire est riche en lipides, plus de 2 fois la teneur d'un régime alimentaire équilibré standard.

Selon l'invention, les termes « **âge adulte** » ou « **animal adulte** » désignent un animal ayant atteint sa maturité sexuelle.

Par les termes « **troubles métaboliques** » on entend une perturbation des processus de métabolisme normal et entraînant le fait que l'organisme ait trop ou bien pas assez des substances essentielles qui sont nécessaires pour rester en bonne santé, ce qui peut conduire à une altération du métabolisme énergétique, une détérioration de l'homéostasie du glucose, une réduction de la sensibilité vis-à-vis de l'insuline (insulinorésistance), un état pro-inflammatoire et/ou une altération du profil lipidique. Dans un mode de réalisation, un trouble métabolique se réfère à l'hyperglycémie post-prandiale. Dans un mode de réalisation, un trouble métabolique se réfère à une hyperglycémie à jeun. Dans un mode de réalisation, un trouble métabolique se réfère à une hyperlipidémie. Dans un mode de réalisation, un trouble métabolique se réfère à une surcharge pondérale. Dans un mode de réalisation, un trouble métabolique se réfère à une surcharge de graisse sous-cutanée ou viscérale. Dans un mode de réalisation, un trouble métabolique se réfère au catabolisme de nutriments ralenti.

Dans un mode de réalisation, un trouble métabolique se réfère à un ensemble d'états métaboliques sélectionnés parmi l'hyperglycémie post-prandiale, l'hyperglycémie à jeun, l'hyperlipidémie, la surcharge pondérale, la surcharge de graisse sous-cutanée, la surcharge de graisse viscérale, le catabolisme de nutriments ralenti.

Dans un mode de réalisation, un trouble métabolique se réfère à un ensemble d'états métaboliques sélectionnés d'un groupe comprenant ou consistant en l'hyperglycémie post-prandiale, l'hyperglycémie à jeun, l'hyperlipidémie, la surcharge pondérale, la surcharge de graisse sous-cutané, la surcharge de graisse viscérale, le catabolisme de nutriments ralenti ou leur combinaison.

Dans un mode de réalisation, un trouble métabolique se réfère à un ensemble d'états métaboliques sélectionnés d'un groupe comprenant ou consistant en l'hyperglycémie post-prandiale, l'hyperglycémie à jeun, l'hyperlipidémie, la surcharge pondérale, la surcharge de graisse sous-cutanée, la surcharge de graisse viscérale ou le catabolisme de nutriments ralenti.

Dans un mode de réalisation, un trouble métabolique se réfère à un ensemble d'états métaboliques sélectionnés d'un groupe comprenant ou consistant en l'hyperglycémie post-prandiale, l'hyperglycémie à jeun, l'hyperlipidémie, la surcharge pondérale, la surcharge de graisse sous-cutanée ou la surcharge de graisse viscérale.

Dans un mode de réalisation, un trouble métabolique se réfère à un ensemble d'états métaboliques sélectionnés d'un groupe comprenant ou consistant en l'hyperglycémie post-prandiale, l'hyperglycémie à jeun, la surcharge pondérale, la surcharge de graisse sous-cutanée ou la surcharge de graisse viscérale.

Par les termes « **empêcher des troubles métaboliques liés à un régime alimentaire déséquilibré à l'âge adulte** » ou « empêcher des troubles métaboliques liés à un régime alimentaire déséquilibré d'un animal à l'âge adulte » on entend la mesure d'une limitation ou d'une réduction de troubles métaboliques, notamment par la mesure de paramètres liés à l'homéostasie du glucose, le métabolisme des lipides et le profil inflammatoire à l'âge adulte entre deux animaux adultes, dont l'un a suivi le traitement lors d'un âge précoce, contrairement à l' autre, comme défini dans les revendications. En effet, selon l'invention, bien que des ScFOS soient administrés à un âge précoce lors de la période de lactation (par exemple pour des porcelets de 5 jours à 21 jours) ou pendant au moins un mois après sevrage (pour les porcelets, le sevrage a lieu entre 21 jours et 48 jours), la prévention de troubles métaboliques est observée à l'âge adulte, c'est-à-dire une longue période après la fin de l'administration des ScFOS. Dans le cas du porc, l'animal est abattu à un âge de 274 jours (39 semaines). Par conséquent, la période entre la fin du traitement et l'observation de la prévention des troubles métaboliques se trouve entre 16 et 31 semaines, c'est-à-dire une période qui est supérieure à la moitié de la durée de la vie du porc. Cet effet est appelé « imprégnation métabolique ». Il était très surprenant qu'un tel effet d'imprégnation métabolique puisse être induit par une fibre prébiotique. En outre, comme toutes les fibres prébiotiques modulent le microbiote intestinal de manière différente, il était également surprenant que les ScFOS modulent spécifiquement le microbiote intestinal et qu'une telle modulation dure pendant toute la vie de l'animal traité.

Selon l'invention, le terme « **âge précoce** » dans l'expression « animal d'âge précoce » on entend un animal juvénile, c'est-à-dire pendant la vie prénatale, l'allaitement, le sevrage et la période post-sevrage dans les limites définies par les revendications. La période post-sevrage est la période pendant laquelle les animaux reçoivent de l'aliment de prédémarrage et de démarrage (c'est-à-dire avant la phase de croissance).

Tel qu'utilisé ici, par le terme « **prévention** » ou « **empêcher** » on entend une réduction du risque de développer un trouble métabolique. Dans un mode de réalisation préféré, les ScFOS sont administrés comme mesure préventive à un sujet présentant une prédisposition vis-à-vis de troubles métaboliques à cause de son environnement ou de sa prédisposition génétique, bien que les symptômes du trouble soient absents ou minimaux. Typiquement, ledit animal, notamment ladite progéniture, peut être un animal monogastrique, préférablement un porcelet et ladite quantité efficace de fructooligosaccharides est administrée :
- à ladite progéniture à de 28 à 56 jours ou de 28 à 77 jours, préférablement de 28 à 56.

Par le terme « quantité efficace » on entend la quantité de FOS, préférablement de ScFOS qui, lors de son administration à un animal, empêchera des troubles métaboliques liés à un régime alimentaire déséquilibré, sans aucun effet secondaire néfaste significatif, par rapport à des animaux non traités.

Tel qu'apprécié dans l'état de la technique, les **quantités efficaces** de FOS pour une utilisation dans la présente invention varieront quelque peu en fonction de l'espèce particulière d'animal ou des conditions de croissance telles que la température et le type d'aliment, et similaires. Pour un cas particulier quelconque, la quantité efficace exacte ou optimale à administrer peut être déterminée par des techniques classiques de titration de dose.

Dans un mode de réalisation, la quantité efficace pour l'animal femelle lors de la gestation et/ou l'allaitement de sa progéniture est de 2 à 20 g, de 4 à 18 g, de 5 à 15 g, de 8 à 12 g de FOS par jour. Dans un mode de réalisation, la quantité efficace pour l'animal femelle lors de la gestation et/ou l'allaitement de sa progéniture est de de 5 à 15 g, de 8 à 12 g de FOS par jour. Dans un mode de réalisation, la quantité efficace pour l'animal femelle lors de la gestation et/ou l'allaitement de sa progéniture est de 8 à 12 g de FOS par jour.

Dans un mode de réalisation, la quantité efficace pour l'animal femelle lors de la gestation et/ou l'allaitement de sa progéniture est environ 5 g, environ 6 g environ 7 g, environ 8 g, environ 9 g, environ 10 g, environ 11 g, environ 12 g, environ 13 g, environ 14 g ou environ 15 g de FOS par jour.

Selon un mode de réalisation, la quantité efficace journalière est repartie à plusieurs administrations par jour. L'homme du métier peut facilement calculer la dose divisée en fonction des prises par jour.

Dans un mode de réalisation, la quantité efficace pour l'animal femelle lors de la gestation et/ou l'allaitement de sa progéniture est de 0.1 à 2%, de 0.1 à 0.6%, de 0.15 à 0.4 ou de 0.15 à 0.3 % w/w par rapport à la composition alimentaire administrée. Dans un mode de réalisation, la quantité efficace pour l'animal femelle lors de la gestation et/ou l'allaitement de sa progéniture est de 0.1 à 0.6%, de 0.15 à 0.4 ou de 0.15 à 0.3 % w/w par rapport à la composition alimentaire administrée. Dans un mode de réalisation, la quantité efficace pour l'animal femelle lors de la gestation et/ou l'allaitement de sa progéniture est de 0.15 à 0.4 ou de 0.15 à 0.3 % w/w, en masse par rapport à la composition alimentaire administrée.Dans un mode de réalisation, la quantité efficace pour la progéniture est de 0.1 à 2 g, de 0.15 à 1 g, de 0.015 à 0.5 g, de 0.15 à 0.5 g de FOS par jour. Dans un mode de réalisation, la quantité efficace pour la progéniture est de 0.3 à 0.5 g, de 0.4 à 0.5 g de FOS par jour.

Dans un mode de réalisation, la quantité efficace pour la progéniture est environ 0.1 g, environ 0.2 g environ 0.3 g, environ 0.4 g, environ 0.5 g, environ 0.6 g, environ 0.7 g, environ 0.8 g, environ 0.9 g ou environ 1 g de FOS par jour.

Selon un mode de réalisation, la quantité efficace journalière est repartie à plusieurs administrations par jour. L'homme du métier peut facilement calculer la dose divisée en fonction des prises par jour.

Dans un mode de réalisation, la quantité efficace pour la progéniture est de 0.05 à 1%, de 0.05 à 0.6%, de 0.1 à 0.4 ou de 0.1 à 0.2 % w/w, en masse par rapport à la composition alimentaire administrée.

Par le terme « **fructo-oligosaccharides** », « fructooligosaccharides » ou « FOS », on entend des bêta-D-fructanes ayant de 2 à 10 unités de fructose, où les unités de fructose sont des chaînes à liaison bêta-2,1-glycosidique, et on trouve une unité unique de D-glucose à l'extrémité terminale. Préférablement, les fructooligosaccharides sont des fructooligosaccharides à chaîne courte. Selon l'invention, les fructooligosaccharides à chaîne courte (scFOS) comprennent de 2 à 8, préférablement de 2 à 4 unités de fructose (GF2, GF3 et GF4). Préférablement, les scFOS selon l'invention comprennent entre 28 et 34%, préférablement 32% (p/p) de GF2, entre 40 et 54%, préférablement 47% (p/p) de GF3 et entre 8 et 16%, préférablement 11% (p/p) de GF4. Les ScFOS sont généralement produits à partir de saccharose par un procédé enzymatique ou de fermentation (JP-A-56-154967, JP-B-59-53834 et JP 61-268190). Les ScFOS sont notamment commercialisés sous la marque de fabrique PROFEED^{®}, ACTILIGHT^{®} ou NUTRAFLORA^{®}. Une méthode analytique de mesure de la teneur en ScFOS est également divulguée dans la technique antérieure par Ouarné *et al.*, 1999, en utilisant une étape d'extraction suivie d'une chromatographie liquide d'échange d'anions associée à une étape d'hydrolyse à l'aide d'invertase. AOAC 999.03 et AOAC 999.08 mentionnent également plusieurs méthodes d'analyse de FOS.

Typiquement, les ScFOS peuvent être administrés en combinaison avec un prébiotique tel que les mannane-oligosaccharides (MOS) ou avec un probiotique (aussi appelé concept symbiotique).

Préférablement, les FOS sont administrés sous la forme d'une poudre ou d'un liquide, ou sont compris dans un complément diététique, ou sont ajoutés directement à l'aliment.

Aussi divulgée, mais pas revendiquée est une méthode de prévention d'une insuffisance d'homéostasie du glucose à l'âge adulte d'un mammifère ou de réduction de la sensibilité aux inflammations à l'âge adulte d'une progéniture issue d'un animal femelle, comprenant l'étape consistant à
- administrer une quantité efficace de fructooligosaccharides à
   i) l'animal femelle lors de la gestation et/ou l'allaitement de ladite progéniture et/ou
   ii) ladite progéniture pendant au moins deux semaines après le sevrage
- empêcher des troubles métaboliques liés à un déséquilibre à un âge adulte de ladite progéniture.

Selon l'invention, par la mesure de « **l'homéostasie du glucose** » on entend l'évaluation de la fonction endocrine intestinale (potentiel de sécrétion de GLP-1) et de la sensibilité du pancréas vis-à-vis du glucose (capacité à sécréter de l'insuline en réponse au bolus de glucose). Selon un mode de réalisation, l'amélioration ou la prévention d'une insuffisance d'homéostasie du glucose se réfèrent à un état de bien-être, de préférence de bien-être métabolique d'après lequel un apport calorique important ou excessif ne déstabilise pas les indices métaboliques tels que la glycémie, la lipidémie ou la charge pondérale. Dans un mode de réalisation, le bien-être métabolique se réfère à un état pendant lequel un apport calorique important ou excessif ne déstabilise pas les indices métaboliques tels que la glycémie, la lipidémie ou la charge pondérale. Dans un mode de réalisation, le bien-être métabolique se réfère à un état pendant lequel un apport calorique important ou excessif ne déstabilise la charge pondérale.

Selon l'invention, par la mesure des « inflammations » on entend les marqueurs pro-inflammatoires dans le plasma et/ou les tissus, par exemple les LPS, l'haptoglobine et les cytokines.

L'invention concerne également la stimulation de la fonction endocrine intestinale et 1 'augmentation de la sensibilité du pancréas vis-à-vis du glucose à l'âge adulte d'une progéniture issue d'un animal femelle, comprenant l'étape consistant à
- administrer une quantité efficace de fructooligosaccharides à ladite progéniture pendant au moins deux semaines après le sevrage
- empêcher des troubles métaboliques liés à un déséquilibre à un âge adulte de ladite progéniture

La présente invention sera illustrée davantage par la description et les dessins supplémentaires suivants, qui se réfèrent aux exemples illustrant.

On doit cependant comprendre que ces exemples ne sont donnés qu'à titre d'illustration de l'invention.

« **Composition nutraceutique** » désigne une composition présentant un avantage physiologique ou offre une protection contre les troubles physiologiques ou l'inconfort. Dans le cadre de la présente invention, les troubles physiologiques sont liées aux troubles métaboliques. Par « **aliment fonctionnel** » ou « alicament », on entend un aliment modifié, ou un ingrédient alimentaire modifié ayant un effet bénéfique ou protecteur contre un désordre ou un inconfort, au-delà des nutriments qu'il contient. La composition nutraceutique ou alimentaire selon l'invention conduit à un bien-être à l'animal adulte suite à l'administration d'une composition de l'invention pendant sa période de vie périnatale, de préférence deux semaines après son sévrage. La composition nutraceutique ou alimentaire selon l'invention conduit à un bien-être à l'animal adulte suite à l'administration d'une composition de l'invention à sa mère pendant la période de la gestation et/ou de l'allaitement.

A chaque fois que les expressions « **par exemple** », « **tel que** », « **y compris** » et similaires sont utilisées dans ce texte, l'expression « et sans limitation » est comprise comme y faisant suite, sauf indication contraire explicite. Par conséquent, « par exemple la production d'éthanol » signifie « par exemple et sans limitation la production d'éthanol ».

« **Environ** » désigne une indication numérique référencée plus ou moins 10% de cette indication numérique référencée. Par exemple, le terme « environ 4 » doit comprendre une plage allant de 3,6 à 4,4. Tous les nombres exprimant des quantités d'ingrédients, de conditions de réaction, etc. utilisés dans la spécification doivent être compris comme étant modifiés dans tous les cas par le terme « environ ». Par conséquent, sauf indication contraire, les paramètres numériques exposés dans ce texte constituent des approximations qui peuvent varier en fonction des propriétés désirées que l'on cherche à obtenir. Au pire, et sans tenter de limiter l'application de la doctrine d'équivalents à la portée de revendications quelconques, chaque paramètre numérique doit être compris à la lumière du nombre de chiffres significatifs et des approches d'arrondi ordinaires

« **Eventuel** » ou « **éventuellement** » signifie que l'évènement ou la circonstance décrit(e) par la suite peut se produire ou non, et que la description comporte des cas où ledit événement ou ladite circonstance se produit et des cas où il ou elle ne se produit pas. Par exemple, l'expression « le milieu peut éventuellement contenir du glucose » signifie que le milieu peut contenir ou non du glucose comme ingrédient et que la description comporte des milieux contenant du glucose ainsi que des milieux ne contenant pas de glucose.

Bien qu'ayant des significations distinctes, par les termes « **comprenant** », « **ayant** », **« contenant** » et « **constitué par »** peuvent être remplacés les uns par les autres dans l'ensemble de la description ci-dessus de l'invention.

Dans le cadre de la présente description, tous les composés, polypeptides et peptides peuvent éventuellement être isolés et/ou purifiés.

L'invention sera davantage évaluée à la vue des exemples et figures suivants.

### BRÈVE DESCRIPTION DES FIGURES

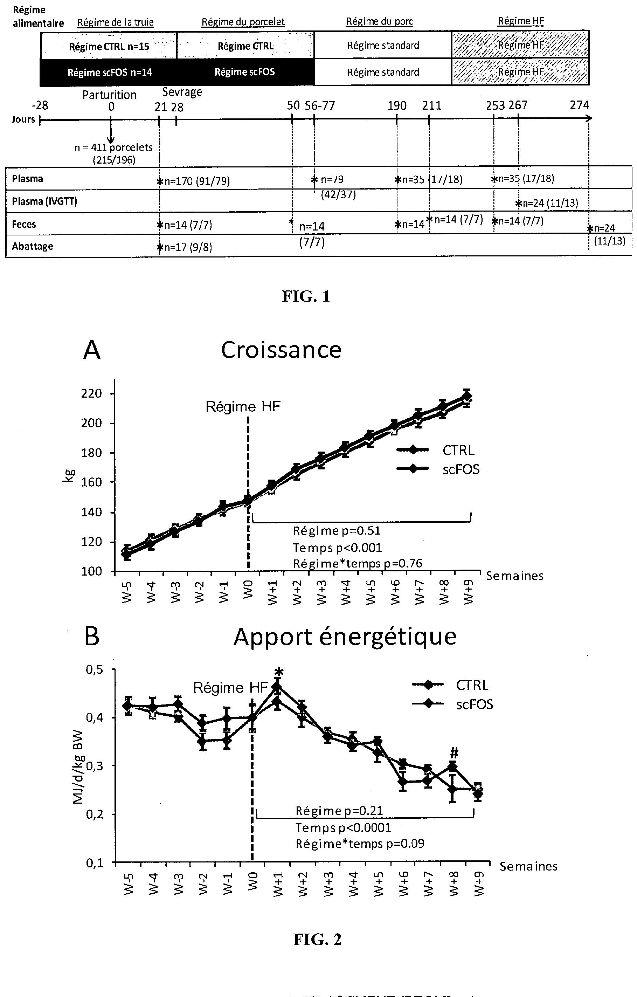

**Figure 1** : Vue d'ensemble du plan d'étude. Vingt-neuf truies et leurs porcelets ont été utilisés dans 2 répétitions (n = 17 dans la 1^{ère} répétition (R1) et n=12 dans la 2^{ème} répétition (R2)). A partir de 28 jours avant le jour présumé de parturition, on a distribué aux truies un régime alimentaire témoin (n = 15, groupe CTRL) ou bien un régime alimentaire complémenté par des fructooligosaccharides à chaîne courte (scFOS) (n = 14, groupe scFOS) jusqu'à la fin de la lactation. Le plasma a été prélevé chez les porcelets de lait (n = 170) à PND 21 et chez les porcs sevrés à PND 77 (n -== 79), PND 190 (n = 35) et PND 253 (n = 35) afin d'analyser l'homéostasie du glucose à des stades différents. Un test de tolérance au glucose IV a été effectué à la fin de l'expérience à PND 267 (n = 24) afin d'évaluer l'homéostasie du glucose. Les matières fécales ont été prélevées à PND 21, PND 190, PND 211 et PND 253 (n = 14/âge de collecte) afin de déterminer la production de SCFA, qui traduit l'activité fermentatrice du microbiote. Dix-sept porcelets ont été sacrifiés à PND 21 afin d'étudier le développement de l'axe entéro-pancréatique, et 24 porcs au stade adulte afin d'évaluer l'adaptation de l'axe entéro-pancréatique à 3 mois du régime HF.
**Figure 2** **:** Poids corporel et apport alimentaire chez des porcs adultes A) Croissance de porcs adultes (n = 17 CTRL et n = 18 scFOS). La croissance s'est accrue significativement au cours du temps (P < 0,001) quel que soit le régime alimentaire périnatal. B) Apport énergétique exprimé en MJ/d/kg de poids corporel (n = 17 CTRL et n = 18 scFOS). L'apport énergétique a diminué au cours du temps (P < 0,001) et on a observé une tendance à une interaction entre le régime alimentaire périnatal et le temps (P = 0,09). Valeurs moyennes ± E.-T.. *: P < 0,05 *vs* CTRL. #: P < 0,10 *vs* CTRL. CTRL, régime alimentaire témoin ; scFOS, régime complémenté par des scFOS ; HF, régime riche en matières grasses.

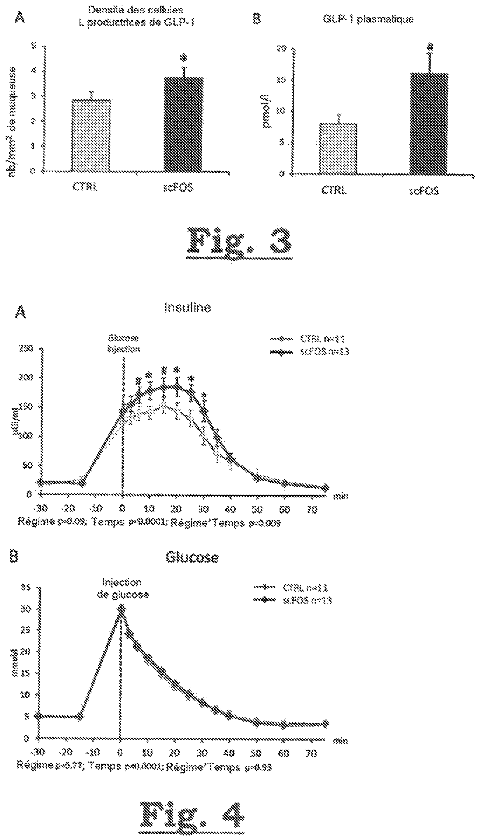

**Figure 3** **:** Densité des cellules L sécrétant GLP-1 dans le caecum et concentration plasmatique en GLP-1 à PND 273 A) Densité en cellules L sécrétant GLP-1 dans le caecum (n = 11 CTRL, et n = 13 scFOS). B) Concentration plasmatique en GLP-1 après un jeûne d'une nuit (n = 11 CTRL et n = 13 scFOS). Valeurs moyennes ± E.-T. ; *: P < 0,05 ; #: P < 0,10. CTRL, régime alimentaire témoin ; scFOS, régime complémenté par des scFOS.
**Figure 4** : Réponses du glucose et de l'insuline à un test de tolérance au glucose IV à PND 267 A) Profil d'insuline (n = 11 CTRL et n = 13 scFOS). B) Profil de glucose (n = 11 CTRL et n = 13 scFOS). Valeurs moyennes ± E.-T. ; *: P < 0,05 vs CTRL ; #: P < 0,10 vs CTRL. CTRL, régime alimentaire témoin ; scFOS, régime complémenté par des scFOS.

### EXEMPLES

La présente invention se comprendra mieux à la lecture des exemples suivants qui illustrent l'invention.

### Matériel et Méthodes

### Animaux, régimes alimentaires et plan d'expérience

Le protocole expérimental a été conçu en conformité avec les législations de l'Union Européenne (directive 86/609/CEE) et de la France (décret 2001-464 29/05/01) pour le soin et l'utilisation des animaux de laboratoire (numéro d'agrément pour l'hébergement d'animaux B-35-275-32). Vingt-neuf truies (Large White × Landrace) et leurs porcelets ((Large White × Landrace) x Piétrain) issus du troupeau expérimental de l'INRA (Saint-Gilles, France) ont été utilisés dans 2 répétitions (n = 17 dans la 1^{ère} répétition (R1) et n = 12 dans la 2^{ème} répétition (R2)). A partir de 28 jours avant le jour présumé de parturition, on a nourri les truies avec un régime alimentaire standard (Cooperl, Lamballe, France) complémenté avec des scFOS (95% de scFOS ayant une longueur de chaîne moléculaire comprise entre 3 et 5 unités monomères, Profeed^{®} P95, Beghin-Meiji, Marckolsheim, France ; groupe scFOS ; n = 14) ou bien des maltodextrines comme témoin (MALDEX, Tereos Syral, Marckolsheim, France ; groupe CTRL ; n = 15) (Figure 1). On a donné aux truies 3 kg.jour⁻¹ d'aliment lors de la gestation et elles ont été nourries *ad libitum* lors de la lactation, conduisant à un apport quotidien d'environ 10 g de scFOS sur les périodes expérimentales de gestation et de lactation, tel que détaillé précédemment (Le Bourgot *et al.*, 2014). Avant sevrage, les porcelets élevés par les truies n'avaient aucun accès à l'alimentation sélective. Au sevrage (jour postnatal (PND) 28), les porcelets ont été nourris *ad libitum* avec un régime alimentaire de démarrage du commerce (Cooperl) complémenté par 0,15% de maltodextrines (CTRL ; n = 42) ou des scFOS (scFOS ; n = 37) selon le régime alimentaire maternel, pendant de 4 à 7 semaines. Ensuite, des porcs ont été choisis aléatoirement afin de poursuivre l'expérience (n = 17 CTRL et n = 18 scFOS) et ont été élevés à l'aide d'un régime alimentaire de croissance du commerce (Cooperl) jusqu'à un âge de 6 mois et un régime alimentaire hautement énergétique et riche en matières grasses (HF), formulé à l'INRA pour fournir 22,6% d'énergie à partir de lipides, jusqu'à un âge de 9 mois (PND 274) (Figure 1). Le poids corporel de la progéniture a été mesuré chaque semaine jusqu'au sevrage puis toutes les deux semaines lors de la période de complémentation. A partir du PND 155 (5 semaines avant l'introduction du régime HF), les porcs ont été pesés chaque semaine jusqu'à la fin de l'expérience. Les porcs ont été suivis quotidiennement en ce qui concerne l'apport alimentaire, ainsi qu'au niveau de fièvres ou de diarrhées. Aucun médicament ou traitement antibiotique n'a été administré sur l'ensemble du protocole expérimental. La composition du régime alimentaire des truies et du régime alimentaire des porcs est fournie dans le Tableau 1 complémentaire.

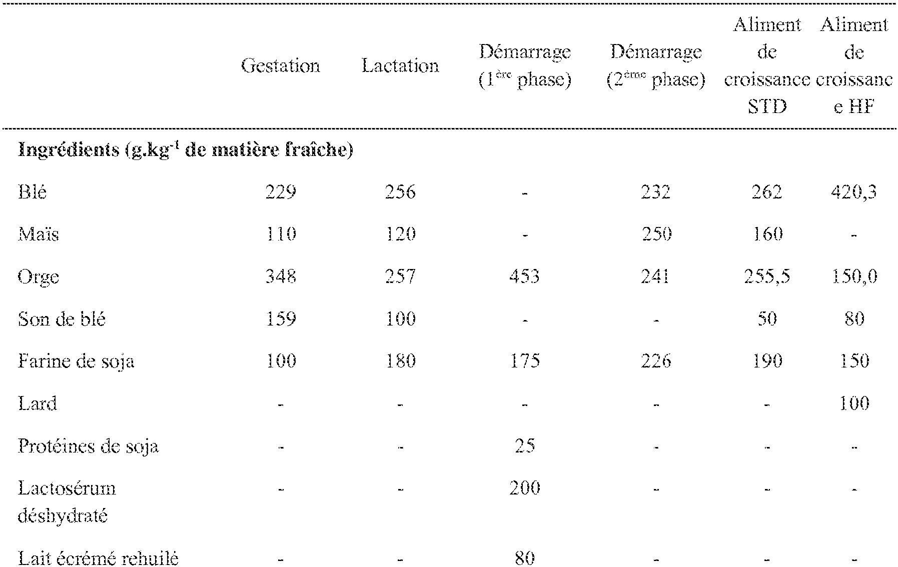

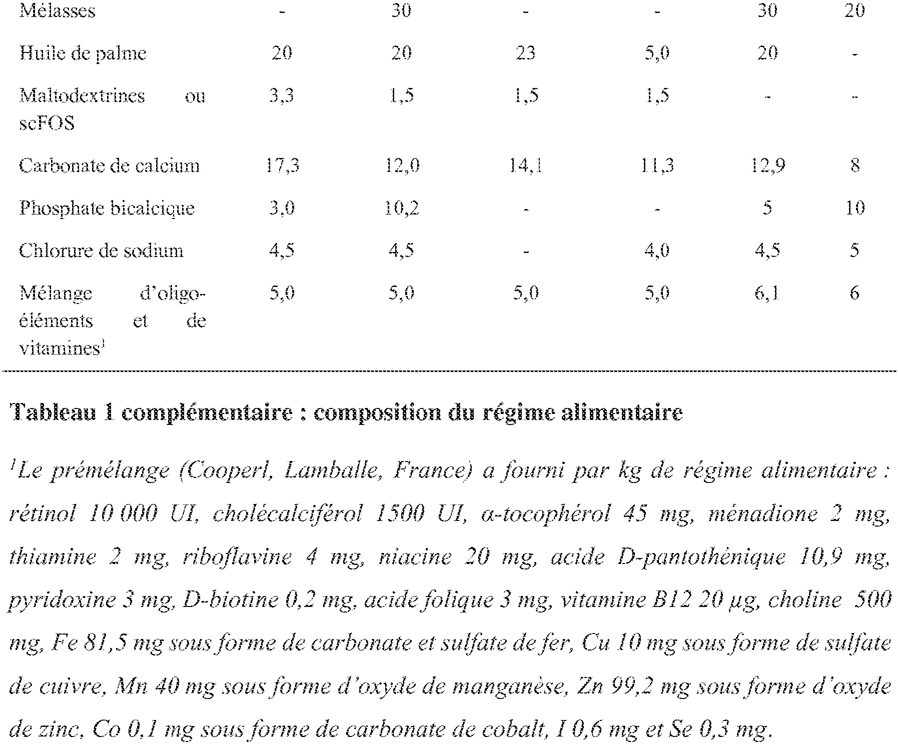

### Collecte d 'échantillons fécaux et sanguins

Des échantillons fécaux et sanguins ont été prélevés à différents stades du développement lors de l'expérience. Des échantillons fécaux ont été prélevés à PND 21, 50, 190, 211 et 253 (n = 7 par groupe alimentaire et par âge) afin de déterminer la production de SCFA, qui traduit l'activité fermentatrice du microbiote (Figure 1). Du sang a été prélevé dans un sous-ensemble de porcelets à PND 21 (n = 91 CTRL et n = 79 scFOS) et après sevrage, à PND 77 (n = 42 CTRL et n = 37 scFOS), PND 190 (n = 17 CTRL et n = 18 scFOS) et PND 253 (n = 17 CTRL et n = 18 scFOS) dans des tubes contenant de l'EDTA. Après centrifugation, les échantillons de plasma ont été stockés à -20°C pour une analyse supplémentaire des concentrations en glucose et en insuline ainsi que des marqueurs inflammatoires.

### Collecte d'échantillons tissulaires

À PND 21 et 274, 17 (n = 9 CTRL et n = 8 scFOS) et 24 (n = 11 CTRL et n = 13 scFOS) porcs respectivement, ont été euthanasiés dans l'abattoir expérimental par étourdissement électrique et exsanguination (Figure 1). Après ouverture de la cavité intestinale, le sang porte a été prélevé (PDN 21) dans des tubes contenant un inhibiteur de la dipeptidyl peptidase IV (DPP-IV) (10 µl/ml de sang, Millipore, Billeria, USA) et de l'EDTA. Des tissus intestinaux issus de l'iléon, du caecum et du côlon (PDN 21 et 274) ont été prélevés, rincés par du PBS froid et stockés à -80°C jusqu'à l'extraction et le dosage de GLP-1. Pour des analyses immunohistochimiques ultérieures, des échantillons de 1 cm³ ont été disséqués à partir du caecum et à partir du corps du pancréas endocrine et fixés dans du paraformaldéhyde à 4%. Un autre échantillon du corps du pancréas a été prélevé et stocké directement à -80°C pour l'extraction d'insuline. Des échantillons du muscle long dorsal, du tissu adipeux viscéral et du tissu adipeux sous-cutané dorsal ont été congelés dans de l'azote liquide et stockés à -80°C jusqu'à une analyse de biologie moléculaire supplémentaire.

### Test de tolérance au glucose intraveineux (IVGTT)

À PND 253, un cathéter a été inséré, sous anesthésie générale, dans une veine jugulaire externe des animaux (n = 11 CTRL et n = 13 scFOS). Une semaine après l'opération chirurgicale, après un jeûne d'une nuit, le test de tolérance au glucose IV (IVGTT) a été mis en oeuvre, consistant en échantillonnages multiples de sang : deux échantillons de base ont été prélevés 30 et 15 min avant injection IV de glucose (0,5 g/kg de poids corporel) et aux temps 0 (juste après la fin de l'injection de glucose), 3, 6, 10, 15, 20, 25, 30, 35, 40, 50, 60 et 75 min après l'injection de glucose. Le sang a été collecté dans des tubes contenant de l'EDTA (ou de l'EDTA plus un inhibiteur de DPP-IV tel que décrit ci-dessus pour les échantillons de base). Après centrifugation, les échantillons de plasma ont été stockés à -20°C (ou -80°C pour les échantillons de base) pour des analyses ultérieures. Les dosages d'insuline et de glucose ont été réalisés sur tous les échantillons de plasma et les dosages de GLP-1 n'ont été réalisés que sur les échantillons de base. La surface incrémentale sous la courbe (AUC) a été calculée sur 75 et 30 min pour le glucose et l'insuline. La réponse d'insuline aiguë (AIR = concentration moyenne en insuline au-dessus des valeurs de base pendant les premières 6 min) et la vitesse de disparition du glucose (K_{G} = pente négative de la droite de régression obtenue avec les valeurs de glucose plasmatique transformées en log de 3 à 30 min, en % par min) ont également été calculées. L'insulino-sensibilité a été évaluée à partir des valeurs de base à jeun du glucose (G_{b}) et de l'insuline (I_{b}) en utilisant l'évaluation du modèle d'homéostasie de la résistance à l'insuline (HOMA-IR= (G_{b}×I_{b})/22,5), et en calculant l'indice S₂ décrit comme étant bien corrélé à l'indice d'insulino-sensibilité obtenu par le test de référence de clamp euglycémique-hyperinsulinémique (Christoffersen *et al.*, 2009) et en prenant en compte l'AUC₀₋₃₀ₘᵢₙ de l'insuline et K_{G}. Les réponses du glucose et de l'insuline ont aussi été intégrées en utilisant le modèle minimal décrit par Bergman (Bergman, 2005) afin d'obtenir l'indice d'insulino-sensibilité S_{I} ainsi que l'indice d'efficacité du glucose S_{G}.

### Dosages d 'hormones, de glucose, de lipides et de marqueurs inflammatoires

Le contenu en insuline a été extrait à partir du pancréas dans 20 ml d'une solution acide d'éthanol (1,5% de HCl 12M, 75% d'éthanol absolu, 23,5% de H₂O). Les concentrations en insuline ont été mesurées dans des extraits de plasma et de pancréas (dilution 1:3000) par une méthode RIA en utilisant de l'insuline porcine iodée (INSULIN-CT, CisbioBioassays, Codolet, France). Le contenu en GLP-1 a été extrait à partir de la muqueuse iléale, du caecum et du côlon par homogénéisation de 1 g de tissu dans 5 ml d'une solution acide d'éthanol (1% de HCl 12M, 74% d'éthanol absolu, 25% de H₂O). La concentration en GLP-1 a ensuite été mesurée dans des échantillons de plasma et intestinaux (dilutions 1:1000, 1:200 et 1:250 pour la muqueuse iléale, le caecum et le côlon respectivement) en utilisant un kit ELISA pour GLP-1 (actif) (Millipore). Le glucose plasmatique, les acides gras non estérifiés (NEFA), les triglycérides, le cholestérol et l'haptoglobine ont été évalués par une méthode spectrophotométrique automatique (Konelab 20i, Thermo Fisher Scientific, Illkirsh, France) en utilisant des kits spécifiques du commerce pour chaque marqueur (Bio Mérieux, Bruz, France). Les LPS plasmatiques ont été mesurés en utilisant un kit ELISA de LPS porcins (MyBiosource, San Diego, USA).

### A nalyses immunohistochimiques

Après fixation pendant 24 h dans du paraformaldéhyde à 4%, les échantillons de pancréas et de caecum ont été cryoprotégés pendant une nuit à 4°C dans du PBS contenant 30% de saccharose, inclus dans le composé OCT (TissueTek, Sakura Finetek Europe B. V., Zoeterwoude, Pays-Bas), congelés dans de l'isopentane et préparés en coupes (10 µm) à l'aide d'un cryostat-microtome. L'analyse immunohistochimique du pancréas a été effectuée tel que décrit précédemment (Blat *et al.*, 2011) afin d'obtenir le nombre et le pourcentage des ilots, et le percentage de tissu endocrine. Les coupes de caecum ont été incubées avec 4% de sérum de cheval normal et du Triton X-100 (0,5%) dans du PBS pendant 30 min. Les coupes ont ensuite été exposées pendant une nuit à un anticorps anti-GLP-1 de lapin (1:500 ; Abcam, Cambridge, R.-U.). Après lavage par du PBS, elles ont été incubées avec un anticorps anti-lapin conjugué à du FITC (1:200 ; Jackson Immuno Research, Baltimore, USA) pendant 3 h. Les coupes ont été de nouveau lavées par du PBS et recouvertes de Vectashield (Vector Labs, Burlingame, USA). On a examiné les coupes à l'aide d'un microscope à fluorescence (Eclipse E400, Nikon Instruments France, Champigny-Sur-Mame, France) fixé à un appareil photographique numérique (Digital Still DXM 1200, Nikon Instruments France). L'ensemble de la coupe de tissu a été numérisée à l'aide d'un scanner de lame porte-objet (Nanozoomer 2,0-RS, Hamamatsu Photonics France SARL, Massy, France). Les fichiers enregistrés ont été analysés à l'aide du logiciel NDPI (Hamamatsu Photonics) pour la détermination du nombre de cellules sécrétant du GLP-1 par zone de muqueuse.

### Extraction d'ARN et PCR quantitative en temps réel (qPCR)

L'ARN total issu d'échantillons de tissu adipeux et de muscle a été extrait par la méthode TRIzol (Invitrogen, France). Les échantillons d'ARN extrait ont été quantifiés à l'aide d'un spectrophotomètre NanoDrop ND-1000 (Thermo Fisher Scientific). La qualité de l'ARN a été vérifiée à l'aide du kit Agilent RNA 6000 Nano et d'un Bioanalyseur Agilent 2100 (Agilent Technologies France, Massy, France). Tous les échantillons répondaient aux critères de qualité. De l'ARN total (2 µg) a été utilisé pour une transcription inverse produisant de l'ADNc, effectuée selon le protocole du fabricant (« High Capacity Complementary DNA Reverse Transcription Kit [Kit de Transcription Inverse d'ADN Complémentaire Haute Capacité] » ; Applied Biosystems). Une PCR en temps réel a été effectuée à l'aide d'une machine StepOnePlus de PCR en temps réel en utilisant un mélange maître SyberGreen (Applied Biosystems) pour la détection. RPL4, GAPDH et YWHAZ ont été utilisés comme gènes domestiques pour les échantillons de muscle et de tissu adipeux viscéral tandis que GAPDH et HPRT1 ont été utilisés comme gènes domestiques pour les échantillons de tissu adipeux sous-cutané selon leur stabilité (Tableau 2 complémentaire). Les gènes d'intérêt ont été analysés selon la méthode 2^{-ΔΔCT}, qui est basée sur la quantification relative du groupe traité (scFOS) par rapport au groupe non traité (CTRL).

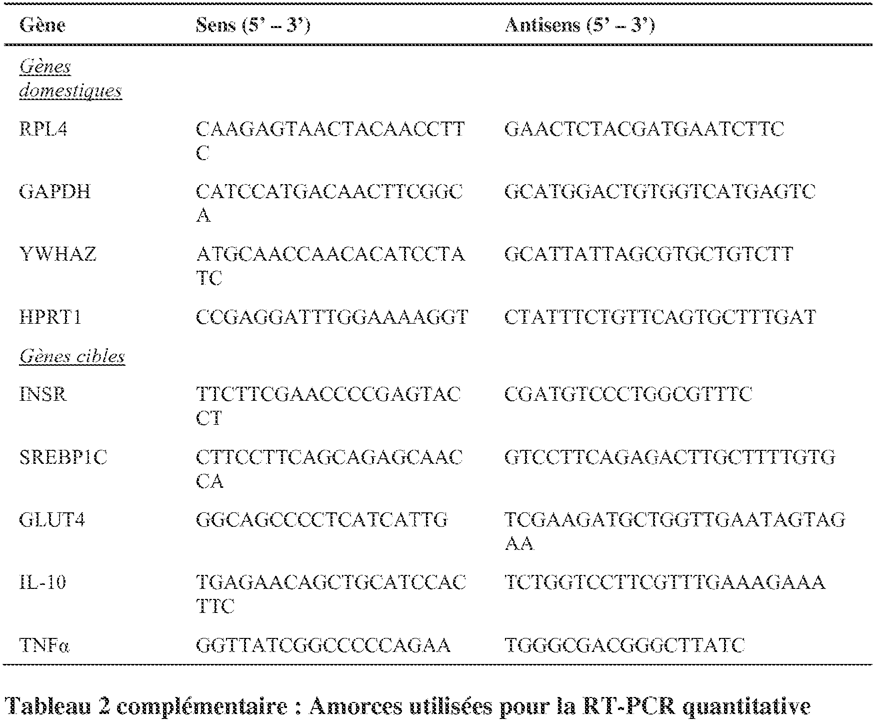

### Mesure des SCFA dans le contenu fécal

Juste après collecte, 1 ml d'une solution d'acide orthophosphorique à 0,5% par g de contenu a été ajouté et les échantillons ont été centrifugés à 1700 g pendant 15 min à 4°C. Les surnageants ont été stockés à -20°C jusqu'à la réalisation du dosage des SCFA par chromatographie gazeuse (Jouany *et al.*, 1981).

### Analyse statistique

Les données ont été soumises à une ANOVA en utilisant « R Core Team » (2013 ; R Foundation for Statistical Computing, Vienne, Autriche ; URL http://www.R-project.org/). Une ANOVA bidirectionnelle avec le régime alimentaire (CTRL vs. scFOS), la répétition (R1 *vs.* R2) et leur interaction comme facteurs a été effectuée pour le poids corporel, les paramètres métaboliques et inflammatoires à chaque âge. Le poids corporel et l'apport énergétique des porcs adultes lors de la période de régime alimentaire HF ont été soumis à une ANOVA avec des mesures répétées comportant le régime alimentaire (CTRL *vs*. scFOS), le temps (semaines), la répétition (R1 vs. R2) et les interactions entre le régime alimentaire et le temps, et le régime alimentaire et la répétition. Lorsque l'effet du régime alimentaire ou l'interaction entre le régime alimentaire et le temps étaient significatifs, les différences entre les groupes alimentaires à chaque temps ont été mesurése davantage par une analyse post-hoc (test de Tukey). De la même façon, les réponses de l'insuline et du glucose vis-à-vis de l'IVGTT ont été analysées par ANOVA avec des mesures répétées comportant le régime alimentaire (CTRL *vs.* scFOS), le temps (minutes après l'injection de glucose), la répétition (R1 *vs.* R2) et les interactions entre le régime alimentaire et le temps, et le régime alimentaire et la répétition, suivie d'une analyse post-hoc (test de Tukey). Les corrélations entre tous les paramètres ont été évaluées à l'aide du test r de Spearman. Toutes les données sont présentées comme la moyenne ± E.-T. La signification statistique a été définie comme une valeur P ≤ 0,05, et la tendance a été rapportée comme une valeur P ≤ 0,10.

### Résultats

### Une complémentation précoce en scFOS n'a pas modifié le poids corporel et l'apport énergétique

Une complémentation précoce en scFOS n'a pas modifié le poids corporel à un stade quelconque du développement (jusqu'à PND 190) (Tableau 1 et Figure 2A). Le régime alimentaire HF n'a pas induit de différences de croissance supplémentaires quelconques entre les porcs scFOS et CTRL (Figure 2A) même si les porcs scFOS avaient tendance à ingérer plus d'énergie pendant la 1^{ère} (P = 0,02) et la 8^{ème} semaine (P = 0,07) du régime alimentaire HF (Figure 2B).

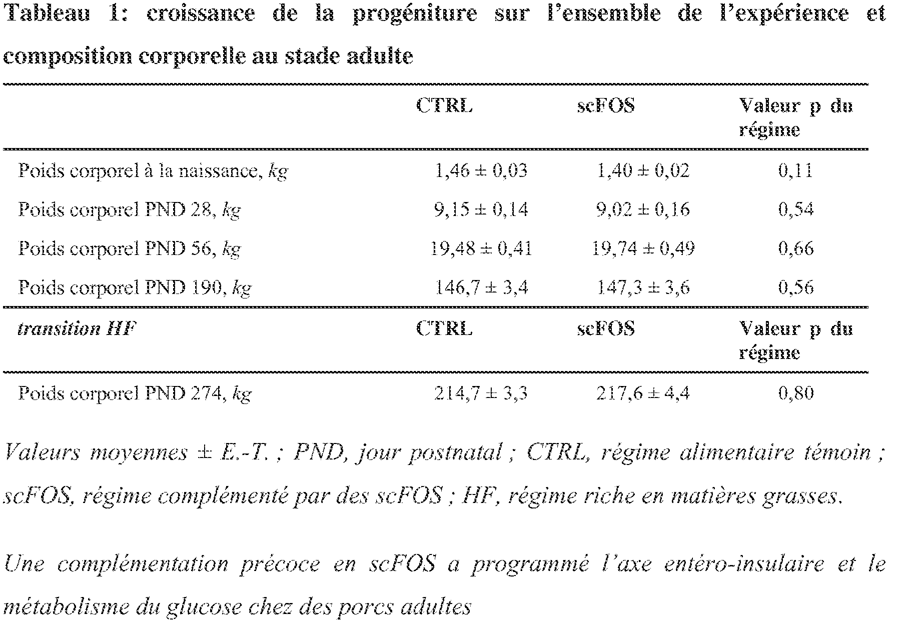

La densité en cellules L sécrétant GLP-1 dans le caecum était supérieure (+33%) dans le groupe scFOS par rapport aux porcs CTRL (P = 0,03 ; Figure 3A). De manière concomitante, la concentration plasmatique en GLP-1 à jeun a eu tendance à augmenter d'environ deux fois dans le groupe scFOS par rapport au groupe CTRL (P = 0,09 ; Figure 3B). De manière intéressante, ces deux paramètres étaient corrélés de manière positive (P = 0,05, R = 0,47). Dans le pancréas, aucune différence n'a été mesurée sur la proportion du tissu endocrine (0,97 ± 0,16% pour CTRL et 0,96 ± 0,09% pour le groupe scFOS) ni sur la teneur en insuline (6,70 ± 1,61 µUI/g pour CTRL et 7,20 ± 1,57 µUI/g pour le groupe scFOS). Cependant, la teneur en insuline pancréatique était corrélée positivement avec la densité en cellules L caecales sécrétant GLP-1 (P = 0,001, R = 0,65).

Les concentrations en insuline et en glucose plasmatiques à jeun n'étaient pas différentes entre les groupes ni avant (PND 190) ni après (PND 253) la période de régime alimentaire HF (Tableau 2), ce n'était pas le cas non plus pour les concentrations en lipides (triglycérides, acides gras libres et cholestérol total ; données non montrées). Lorsque l'homéostasie du glucose a été provoquée par un injection intraveineuse d'un bolus de glucose (IVGTT), les animaux scFOS ont sécrété plus d'insuline que les animaux CTRL sans différence du profil de glucose (Figure 4). Les indices calculés à partir des profils d'insuline et de glucose ont conduit à une tendance à une AUC d'insuline supérieure entre 0 et 30 min après injection de glucose chez des porcs scFOS (P = 0,07), ainsi qu'une réduction de S₂ (P = 0,09) et une réduction significative des indices S_{I} (P = 0,02) (Tableau 2). Une corrélation négative a été établie entre AUC₀₋₃₀ₘᵢₙ du glucose et les cellules L caecales produisant GLP-1 (P = 0,001, R = -0,62).

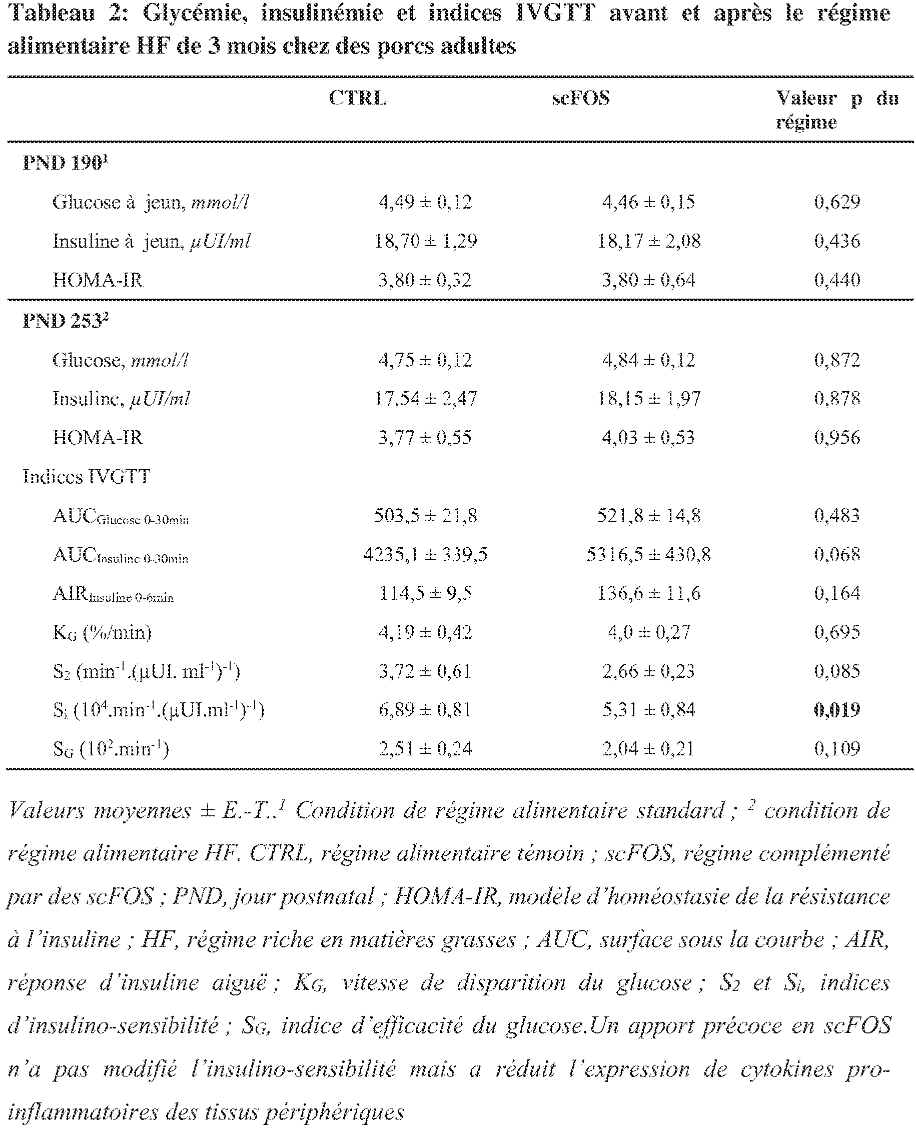

Aucun parmi les gènes étudiés représentatif de l'insulino-sensibilité dans le muscle et les tissus adipeux n'a présenté de différences au niveau des taux d'ARNm entre les groupes CTRL et scFOS à PND 273 (Tableau 3). L'expression de TNFa et d'IL-10 n'était pas non plus différente entre les animaux scFOS et CTRL dans le tissu adipeux sous-cutané, mais l'expression de TNFα a été significativement réduite dans le tissu adipeux viscéral de porcs scFOS par rapport aux porcs CTRL (P = 0,02 ; Tableau 3).

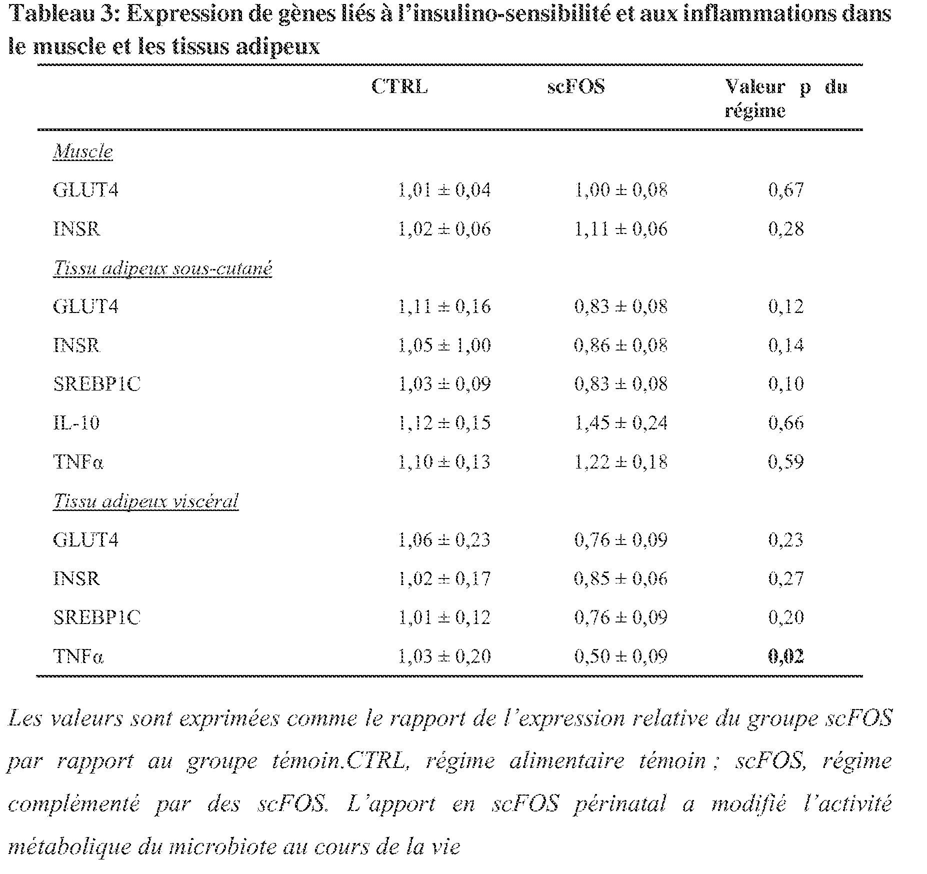

La production totale de SCFA a augmenté chez les porcelets de lait (PND 21) dont les mères ont reçu une complémentation en scFOS (P = 0,001 ; Tableau 5), plus particulièrement en acétate, propionate, valérate et caproate. Après sevrage, plus aucune différence n'a été observée, ni à la fin de la complémentation en scFOS (PND 50) ni à PND 190 avec le régime standard. Trois semaines après le début du régime HF, les porcs scFOS ont eu tendance à présenter une concentration supérieure en acétate dans les fèces (CTRL-HF: 52,3 ± 3,3 mmol/kg et scFOS-HF: 67,2 ± 7,5 mmol/kg ; P = 0,09) qui n'a pas persisté, sans différence entre les groupes 6 semaines plus tard (Tableau 5).

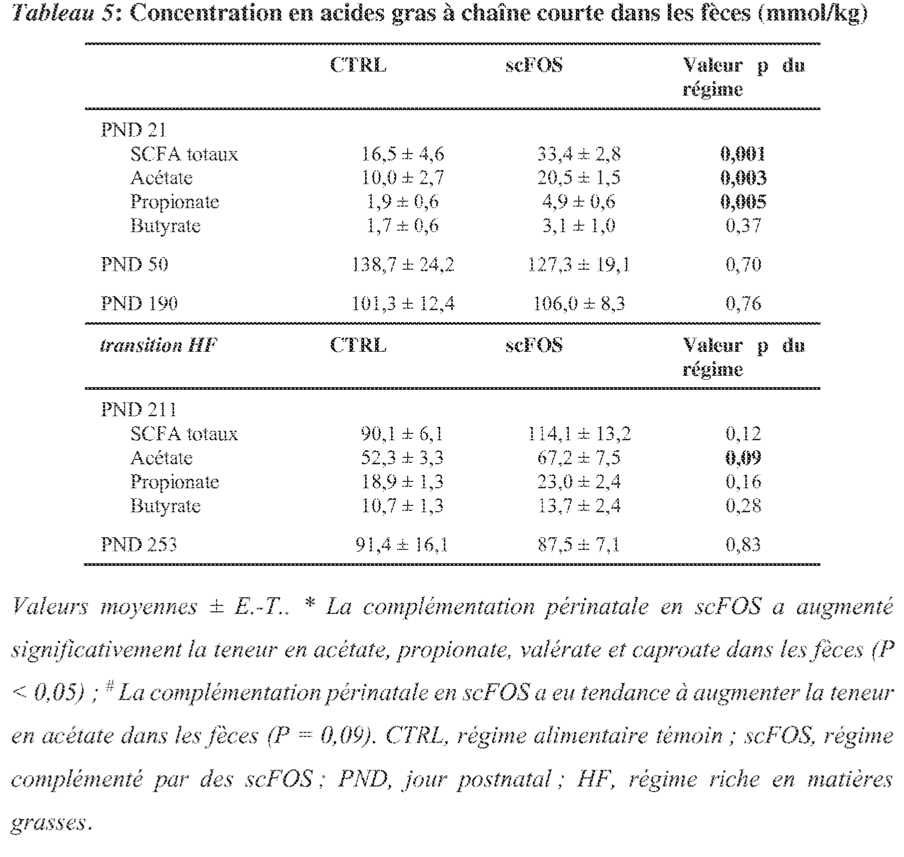

### Discussion

Une accumulation d'indications suggère que la nutrition lors de la vie foetale et postnatale précoce programme le développement microbien, métabolique et immunitaire chez la progéniture, avec des conséquences ultérieures sur sa sensibilité vis-à-vis de maladies métaboliques. La programmation du pancréas est bien documentée, mais on sait peu de choses sur l'imprégnation de la fonction endocrine intestinale, malgré un nombre croissant de données suggérant que l'intestin est sujet à une telle imprégnation nutritionnelle. Notre objectif consistait à déterminer si une complémentation périnatale avec des prébiotiques, connus pour équilibrer de manière correcte le microbiote intestinal, améliorerait la physiologie intestinale, l'axe entéro-insulaire et l'homéostasie du glucose chez la progéniture adulte afin de limiter le développement de troubles métaboliques et/ou inflammatoires induits par un régime alimentaire déséquilibré, en utilisant des porcs classiques comme modèle humain. Nous avons montré qu'une complémentation périnatale par des scFOS programme la fonction entéro-endocrine avec une sensibilité accrue du pancréas vis-à-vis du glucose de manière dépendante de GLP-1, ainsi que la résistance aux inflammations, qui peuvent être bénéfiques afin de lutter contre un régime alimentaire déséquilibré à l'âge adulte.

Le schéma de croissance n'a pas été affecté par la complémentation périnatale par des scFOS, ni non plus le gain de poids corporel lors de la provocation par HF, même si les porcs scFOS ont eu tendance à ingérer plus d'énergie. Les deux groupes ont régulé leur apport énergétique après la première semaine de la transition vers le régime alimentaire HF et n'ont pas développé d'hyperphagie. En effet, il n'y avait aucun effet sur l'état inflammatoire systémique ni sur l'insulino-sensibilité de tissus périphériques. De manière intéressante, même s'il était plus lourd, le tissu viscéral des porcs scFOS a exprimé moins de TNFα par rapport au groupe CTRL. Hallam *et al.* ont montré une réduction du pourcentage de graisse corporelle totale chez des rats adultes soumis à un régime alimentaire riche en matières grasses et riche en saccharose (HFHS) dont les mères avaient été complémentées par des prébiotiques (Hallam & Reimer, 2013). Cette contradiction peut s'expliquer par la dose supérieure en prébiotiques et la période différente de complémentation utilisées dans leur étude (21,6% pendant l'ensemble de la gestation) par rapport aux nôtres (0,33% pendant le dernier tiers de la gestation et 0,15% pour la lactation et le post-sevrage) ainsi que la composition différente du régime alimentaire à l'âge adulte (HF *vs* HFHS) (Hallam & Reimer, 2013).

Une tendance vers une concentration supérieure en GLP-1 à jeun a été observée chez des porcs adultes ayant reçu une complémentation précoce en scFOS, en association avec une densité en cellules L sécrétant GLP-1 accrue significative dans le caecum. Ces données ont indiqué une capacité accrue de la progéniture adulte scFOS à sécréter du GLP-1. Des rats adultes, après une complémentation périnatale en oligofructose, n'ont pas présenté de changements du taux postprandial en GLP-1 mais ont présenté une augmentation de la concentration en peptide inhibiteur gastrique, une autre hormone de type incrétine, sécrétée par des cellules K plus proximales (Hallam & Reimer, 2013). En outre, lors d'une ingestion directement par des adultes en bonne santé ou des adultes présentant des conditions métaboliques (régime alimentaire HF ou individus obèses), les prébiotiques augmentent le nombre de cellules L intestinales produisant GLP-1, la teneur en GLP-1 et les taux circulants de GLP-1 chez l'homme, le rat et la souris (Cani *et al.*, 2004 ; Cani *et al.*, 2006 ; Delmee *et al.*, 2006 ; Cani *et al.*, 2007 ; Cani *et al.*, 2009 ; Everard *et al.*, 2011 ; Kaji *et al.*, 2011 ; Parnell & Reimer, 2012), ce qui peut, à son tour, améliorer leur état métabolique (Everard & Cani, 2013). Les SCFA produits par le microbiote après un apport en scFOS augmentent la production et la libération de GLP-1 par les cellules L entéro-endocrines dans l'intestin et favorisent la différentiation des cellules L dans les cryptes (Kaji *et al.*, 2011). Dans notre étude, une complémentation périnatale par des scFOS a présenté des effets sur les cellules L similaires à un apport direct de prébiotiques sept mois après l'arrêt de l'apport en prébiotiques. L'une des hypothèses est le maintien au cours du temps d'un microbiote favorable, notamment avec une activité fermcntatrice élevée, établie au cours du début de la vie. Ceci est supporté par une corrélation positive observée entre la production totale de SCFA et la concentration plasmatique en GLP-1. La teneur en SCFA totaux a été accrue dans les fèces de porcelets de lait scFOS (PND 21), révélant une modulation potentielle du microbiote, la production de métabolites dépendant de la composition du microbiote dans la lumière intestinale et de ses substrats. En relation avec ces modifications précoces des métabolites, la complémentation maternelle en scFOS a induit une amélioration de la tolérance au glucose chez des porcelets de lait, la quantité d'insuline requise pour retourner à la glycémie de base après allaitement ayant diminué. Aucun changement n'a été observé sur la densité des cellules L intestinales, mais une tendance à une diminution de la concentration plasmatique en en GLP-1 a été montrée chez des porcelets de lait scFOS. Nous avons précédemment montré qu'une complémentation maternelle en scFOS (plus tard dans la gestation et ensemble de la lactation) a induit une production accrue en butyrate dans le contenu du côlon un mois après sevrage (Le Bourgot *et al.*, en révision) et une activité fermentatrice accrue du microbiote de porcelets âgés de trois mois (Le Bourgot *et al.*, 2014). Ici, nous avons observé une adaptation différente au régime alimentaire HF, avec plus d'acétate produit chez des porcs adultes issus du groupe scFOS trois semaines après le début du régime alimentaire déséquilibré, suggérant que des différences de composition du microbiote peuvent persister jusqu'à l'âge adulte. Par conséquent, une modulation du microbiote tôt dans la vie, par un apport maternel en prébiotiques a induit des modifications persistantes avec une augmentation de *Bifidobacterium spp.* et *Clostridium coccoides* chez une progéniture de rats adultes (Hallam *et al.*, 2014). Par conséquent, nos données ont démontré une programmation de la fonction endocrine intestinale probablement médiée par l'établissement précoce d'un microbiote bénéfique et son activité métabolique.

Une fois libéré à partir des cellules L intestinales, GLP-1 stimule la sécrétion d'insuline et exerce un effet trophique sur le pancréas par la régulation de la prolifération et de la différentiation des cellules des ilots (Baggio & Drucker, 2007). Aucune différence n'a été observée au niveau de l'anatomie pancréatique et de la teneur en insuline entre les porcs adultes scFOS et CTRL mais le groupe scFOS a eu tendance à sécréter plus d'insuline en réponse à un stimulus de glucose IV sans modification du profil de glucose. Cette libération accrue d'insuline a fait ressortir une sensibilité supérieure du pancréas vis-à-vis du glucose chez les porcs scFOS, car ils ne présentaient aucune résistance à l'insuline dans leurs tissus périphériques. Ceci pourrait être attribué à leur concentration basale supérieure en GLP-1, tel que suggéré par Chan *et al.* qui ont démontré que des doses croissantes de GLP-1 stimulaient la libération d'insuline lors d'un IVGTT chez la souris (Chan *et al.*, 2011). Il y avait une corrélation positive entre la densité en cellules L sécrétant GLP-1 et la teneur en insuline pancréatique, soulignant le rôle de GLP-1 sur la capacité de libération de l'insuline du pancréas dans notre étude. Ceci était conforme à des données chez des souris diabétiques nourries avec un régime riche en matières grasses, chez lesquelles une complémentation en oligofructose pendant quatre semaines était efficace pour réduire la glycémie à jeun et postprandiale en association avec des taux accrus en insuline plasmatique et pancréatique, de manière dépendante de GLP-1 via une augmentation de la concentration plasmatique en GLP-1 et de la teneur en GLP-1 total dans le côlon proximal (Cani *et al.*, 2006). Dans notre étude, nous n'avons pas observé de changements du profil de glucose lors de l'IVGTT, mais la corrélation négative observée entre AUC_{Glucose} et les cellules L productrices de GLP-1, ont supporté le rôle de la fonction endocrine intestinale dans la régulation de l'homéostasie du glucose.

Une activité accrue d'IAP peut favoriser la réduction chronique des concentrations plasmatiques en LPS, et réduire ainsi l'endotoxémie métabolique, qui contribue aux troubles métaboliques associés à un régime alimentaire HF (Everard & Cani, 2013). Bien qu'il n'y cût aucune différence significative de LPS plasmatiques, les valeurs numériques étaient inférieures de 37% chez les porcs scFOS, et de nouveau, l'expression de TNFα dans leur tissu adipeux viscéral était significativement réduite par rapport aux porcs CTRL. Par conséquent, Hallam *et al.* ont montré une réduction des LPS plasmatiques chez des rats adultes soumis à une complémentation en prébiotiques tôt dans la vie, liée à une abondance supérieure en *Bifidobacterium spp.* suggérée comme étant médiée par les oligosaccharides du lait (Hallam *et al.*, 2014). Dans notre étude, nous avons observé une composition différente en macronutriments du lait de la truie avec une complémentation maternelle en scFOS qui pourrait contribuer à moduler la composition et le métabolisme du microbiote colonisant l'intestin néonatal et par conséquent leurs effets à long terme (Hallam *et al.*, 2014). On a montré que l'expression d'IAP était contrôlée par le microbiote intestinal (Bates *et al.*, 2007), ainsi que la libération de sIgA dans la lumière intestinale (Ohland & Jobin, 2015), supportant une persistance potentielle de la modulation du microbiote induite par une complémentation périnatale par des scFOS à l'âge adulte.

Les voies régulatrices, y compris les modifications épigénétiques, ne peuvent être exclues même si leur étude dépassait l'objectif de notre étude. En effet, les changements épigénétiques se produisant lors du développement foetal et postnatal conduisent à la mise en forme des tissus et des organes qui peuvent affecter la santé de l'enfant avec des conséquences possibles à l'âge adulte. Le microbiote intestinal exerce un impact majeur sur l'épigénome de l'hôte de par sa composition et de par la quantité importante de métabolites produits tels que le folate, le butyrate et l'acétate (Mischke & Plosch, 2013).

Globalement, notre étude a démontré, pour la première fois, que la fonction endocrine intestinale et la sensibilité vis-à-vis des inflammations chez des porcs adultes, sont soumises à une imprégnation nutritionnelle pouvant participer à l'imprégnation métabolique. Une complémentation périnatale par des scFOS, connus pour équilibrer convenablement le microbiote de manière durable, pourrait constituer une stratégie intéressante pour empêcher les troubles métaboliques et la sensibilité vis-à-vis des inflammations plus tard dans la vie, notamment lors d'une confrontation à un environnement nutritionnel délétère. Des études supplémentaires sur les spécifiques interactions entre l'apport périnatal en scFOS et le microbiote sont toujours justifiées

### RÉFÉRENCES

Ajslev TA, Andersen CS, Gamborg M, Sorensen TI & Jess T. (2011). Childhood overweight after establishment of the gut microbiota: the rôle of delivery mode, pre-pregnancy weight and early administration of antibiotics. Int J Obes (Lond) 35, 522-529.
Baggio LL & Drucker DJ. (2007). Biology of incretins: GLP-1 and GIP. Gastroenterology 132, 2131-2157.
Bates JM, Akerlund J, Mittge E & Guillemin K. (2007). Intestinal alkaline phosphatase detoxifies lipopolysaccharide and prevents inflammation in zebrafish in response to the gut microbiota. C'ell Host Microbe 2, 371-382.
Bedford Russell AR & Murch SH. (2006). Could peripartum antibiotics hâve delayed health conséquences for the infant? Bjog 113, 758-765.
Bergman RN. (2005). Minimal model: perspective from 2005. Horm Res 64 Suppl 3, 8-15.
Bertin E, Gangnerau MN, Bailbe D & Portha B. (1999). Glucose metabolism and beta-cell mass in adult offspring of rats protein and/or energy restricted during the last week of pregnancy. Am J Physiol 277, E11-17.
Boudry G, Jamin A, Chatelais L, Gras-Le Guen C, Michel C & Le Huerou-Luron I. (2013). Dietary protein excess during néonatal life alters colonie microbiota and mucosal response to inflammatory mεdiators later in life in female pigs. J Nutr 143, 1225-1232.
Boullier S, Tanguy M, Kadaoui KA, Caubet C, Sansonetti P, Corthesy B & Phalipon A. (2009). Secretory IgA-mediated neutralization of Shigella flexneri prevents intestinal tissue destruction by down-regulating inflammatory circuits. J Immunol 183, 5879-5885.
Cani PD, Dewever C & Delzenne NM. (2004). Inulin-type fructans modulate gastrointestinal peptides involved in appetite régulation (glucagon-like peptide-1 and ghrelin) in rats. Br J Nutr 92, 521-526.
Cani PD, Hoste S, Guiot Y & Delzenne NM. (2007). Dietary non-digestible carbohydrates promote L-cell différentiation in the proximal colon of rats. Br J Nutr 98, 32-37.
Cani PD, Knauf C, Iglesias MA, Drucker DJ, Delzenne NM & Burcelin R. (2006). Improvement of glucose tolérance and hepatic insulin sensitivity by oligofructose requires a functional glucagon-like peptide 1 receptor. Diabètes 55, 1484-1490.
Cani PD, Possemiers S, Van de Wiele T, Guiot Y, Everard A, Rottier O, Geurts L, Naslain D, Neyrinck A, Lambert DM, Muccioli GG & Delzenne NM. (2009). Changes in gut microbiota control inflammation in obese mice through a mechanism involving GLP-2-driven improvement of gut permeability. Gut 58, 1091-1103.
Chan HM, Jain R, Ahren B, Pacini G & D'Argenio DZ. (2011). Effects of increasing doses of glucagon-like peptide-1 on insulin-releasing phases during intravenous glucose administration in mice. Am J Physiol Regul Integr Comp Physiol 300, R1126-1133.
Chatelais L, Jamin A, Gras-Le Guen C, Lalles JP, Le Huerou-Luron I & Boudry G. (2011). The level of protein in milk formula modifies ileal sensitivity to LPS later in life in a piglet model. PLoS One 6, e19594.
Christoffersen B, Ribel U, Raun K, Golozoubova V & Pacini G. (2009). Evaluation of different methods for assessment of insulin sensitivity in Gottingen minipigs: introduction of a new, simpler method. Am J Physiol Regul Integr Comp Physiol 297, R1195-1201.
Cox LM, Yamanishi S, Sohn J, Alekseyenko AV, Leung JM, Cho I, Kim SG, Li H, Gao Z, Mahana D, Zarate Rodriguez JG, Rogers AB, Robine N, Loke P & Blaser MJ. (2014). Altering the intestinal microbiota during a critical developmental window has lasting metabolic conséquences. Cell 158, 705-721.
De Filippo C, Cavalieri D, Di Paola M, Ramazzotti M, Poullet JB, Massart S, Collini S, Pieraccini G & Lionetti P. (2010). Impact of diet in shaping gut microbiota revealed by a comparative study in children from Europe and rural Africa. Proc Natl Acad Sci U S A 107, 14691-14696.
Delmee E, Cani PD, Gual G, Knauf C, Burcelin R, Maton N & Delzenne NM. (2006). Relation between colonie proglucagon expression and metabolic response to oligofructose in high fat diet-fed mice. Life Sci 79, 1007-1013.
Everard A & Cani PD. (2013). Diabètes, obesity and gut microbiota. Best Pract Res Clin Gastroenterol 27, 73-83.
Everard A, Lazarevic V, Derrien M, Girard M, Muccioli GG, Neyrinck AM, Possemiers S, Van Holle A, Francois P, de Vos WM, Delzenne NM, Schrenzel J & Cani PD. (2011). Responses of gut microbiota and glucose and lipid metabolism to prebiotics in genetic obese and diet-induced leptin-resistant mice. Diabètes 60, 2775-2786.
Fanca-Berthon P, Michel C, Pagniez A, Rival M, Van Seuningen I, Darmaun D & Hoebler C. (2009). Intrauterine growth restriction alters postnatal colonie barricr maturation in rats. Pediatr Res 66, 47-52.
Fujiwara R, Watanabe J & Sonoyama K. (2008). Assessing changes in composition of intestinal microbiota in neonatal BALB/c mice through cluster analysis of molecular markers. Br J Nutr 99, 1174-1177.
Garofano A, Czemichow P & Breant B. (1998). Beta-cell mass and prolifération following late fetal and early postnatal malnutrition in the rat. Diabetologia 41, 1114-1120.
Garofano A, Czemichow P & Breant B. (1999). Effect of ageing on beta-cell mass and function in rats malnourished during the perinatal period. Diabetologia 42, 711-718.
Gluckman PD, Hanson MA & Beedle AS. (2007). Early life events and their conséquences for later disease: a life history and evolutionary perspective. Am J Hum Biol 19, 1-19.
Godfrey KM, Gluckman PD & Hanson MA. (2010). Developmental origins of metabolic disease: life course and intergenerational perspectives. Trends Endocrinol Metab 21, 199-205.
Hallam MC, Barile D, Meyrand M, German JB & Reimer RA. (2014). Maternal high-protein or high-prebiotic-fiber diets affect maternai milk composition and gut microbiota in rat dams and their offspring. Obesity (Silver Spring) 22, 2344-2351.
Hallam MC & Reimer RA. (2013). A maternai high-protein diet prédisposes female offspring to increased fat mass in adulthood whereas a prebiotic fibre diet decreases fat mass in rats. Br J Nutr 110, 1732-1741.
Jouany JP, Zainab β, Senaud J, Groliere CA, Grain J & Thivend P. (1981). Rôle of the rumen ciliate protozoa polyplastron-multivesiculatum, entodinium sp and isotricha-prostoma in the digestion of a mixed diet in sheep. Reproduction Nutrition Development 21,871-884.
Kaji 1, Karaki S, Tanaka R & Kuwahara A. (2011). Density distribution of free fatty acid receptor 2 (FFA2)-expressing and GLP-1-producing enteroendocrine L cells in human and rat lower intestine, and increased cell numbers after ingestion of fructooligosaccharide. J Mol Histol 42, 27-38.
Lalles JP. (2010). Intestinal alkaline phosphatase: multiple biological roles in maintenance of intestinal homeostasis and modulation by diet. Nutr Rev 68, 323-332.
Le Bourgot C, Ferret-Bernard S, Le Normand L, Savary G, Menendez-Aparicio E, Blat S, Appert-Bossard E, Respondek F & Le Huerou-Luron I. (2014). Maternai short-chain fructooligosaccharide supplementation influences intestinal immune system maturation in piglets. PLoS One 9, e107508.
Mischke M & Plosch T. (2013). More than just a gut instinct-the potential interplay between a baby's nutrition, its gut microbiome, and the epigenome. Am J Physiol Regul Integr Comp Physiol 304, R1065-1069.
Mourot J. (2004). Contrai of the adiposity in breeding animais. Bulletin de l'Académie Vétérinaire de France 157, 29-34.
Ohland CL & Jobin C. (2015). Microbial activities and intestinal homeostasis: A délicate balance between health and disease. Cell Mol Gastroenterol Hepatol 1, 28-40.
Pan XD, Chen FQ, Wu TX, Tang HG & Zhao ZY. (2009). Prebiotic oligosaccharides change the concentrations of short-chain fatty acids and the microbial population of mouse bowel. J Zhejiang Univ Sci β 10, 258-263.
Parnell JA & Reimer RA. (2012). Prebiotic fibres dose-dependently increase satiety hormones and alter Bacteroidetes and Firmicutes in lean and obese JCR:LA-cp rats. Br J Nutr 107, 601-613.
Penders J, Thijs C, Vink C, Stelma FF, Snijders B, Kummeling I, van den Brandt PA & Stobberingh EE. (2006). Factors influencing the composition of the intestinal microbiota in early infancy. Pediatrics 118, 511-521.
Reinhardt C, Reigstad CS & Backhed F. (2009). Intestinal microbiota during infancy and its implications for obesity. J Pediatr Gastroenterol Nutr 48, 249-256.
Respondek F, Gerard P, Bossis M, Boschat L, Bruneau A, Rabot S, Wagner A & Martin JC. (2013). Short-chain fructo-oligosaccharides modulate intestinal microbiota and metabolic parameters of humanized gnotobiotic diet induced obesity mice. PLoS One 8, e71026.
Roberfroid M, Gibson GR, Hoyles L, McCartney AL, Rastall R, Rowland I, Wolvers D, Watzl B, Szajewska H, Stahl B, Guamer F, Respondek F, Whelan K, Coxam V, Davicco MJ, Leotoing L, Wittrant Y, Delzenne NM, Cani PD, Neyrinck AM & Meheust A. (2010). Prebiotic effects: metabolic and health benefits. Br J Nutr 104 Suppl 2, S1-63.
Roura E, Koopmans SJ, Lalles JP, Le Huerou-Luron I, de Jager N, Schuurman T & Val-Laillet D. (2016). Critical review evaluating the pig as a model for human nutritional physiology. Nutr Res Rev 29, 60-90.
Schokker D, Zhang J, Vastenhouw SA, Heilig HG, Smidt H, Rebel JM & Smits MA. (2015). Long-lasting effects of early-life antibiotic treatment and routine animal handling on gut microbiota composition and immune system in pigs. PLoS One 10, e0116523.
Swanson KS, Grieshop CM, Flickinger EA, Bauer LL, Chow J, Wolf BW, Garleb KA & Fahey GC, Jr. (2002). Fructooligosaccharides and Lactobacillus acidophilus modify gut microbial populations, total tract nutrient digestibilities and fecal protein catabolite concentrations in healthy adult dogs. J Nutr 132, 3721-3731.
Tolhurst G, Heffron H, Lam YS, Parker HE, Habib AM, Diakogiannaki E, Cameron J, Grosse J, Reimann F & Gribble FM. (2012). Short-chain fatty acids stimulate glucagon-like peptide-1 sécrétion via the G-protein-coupled receptor FFAR2. Diabètes 61, 364-371.
Trasande L, Blustein J, Liu M, Corwin E, Cox LM & Blaser MJ. (2013). Infant antibiotic exposures and early-life body mass. Int J Obes (Lond) 37, 16-23.
Van X, Huang Y, Wang H, Du M, Hess BW, Ford SP, Nathanielsz PW & Zhu MJ. (2011). Maternai obesity induces sustained inflammation in both fetal and offspring large intestine of sheep. Inflamm Bowel Dis 17, 1513-1522.

## Revendications

1. Fructooligosaccharides (FOS) pour leur utilisation à un âge précoce d'un animal dans la prévention des troubles métaboliques liés à un régime alimentaire déséquilibré à l'âge adulte ;
lesquels troubles métaboliques étant sélectionnés d'un groupe comprenant ou consistant en l'hyperglycémie post-prandiale, l'hyperglycémie à jeun, l'hyperlipidémie, la surcharge pondérale, la surcharge de graisse sous-cutanée, la surcharge de graisse viscérale, le catabolisme de nutriments ralenti ou leur combinaison ; et
lesdits fructooligosaccharides étant administrés audit animal d'âge précoce entre 2 et 9 semaines après sevrage.

2. Fructooligosaccharides (FOS) pour leur utilisation selon la revendication **1,** lesdits FOS étant administrés en une quantité efficace à
i) un animal femelle lors de la gestation et/ou l'allaitement de sa progéniture et à
ii) ladite progéniture pendant au moins deux semaines après le sevrage.

3. Fructooligosaccharides (FOS) pour leur utilisation selon la revendication **1** ou la revendication **2,** lesquels troubles métaboliques étant sélectionnés d'un groupe comprenant ou consistant l'hyperglycémie post-prandiale, l'hyperglycémie à jeun, l'hyperlipidémie, la surcharge pondérale, la surcharge de graisse sous-cutanée ou la surcharge de graisse viscérale.

4. Fructooligosaccharides (FOS) pour leur utilisation selon la revendication **3,** lesquels troubles métaboliques étant sélectionnés d'un groupe comprenant ou consistant en l'hyperglycémie post-prandiale, l'hyperglycémie à jeun, la surcharge pondérale, la surcharge de graisse sous-cutanée ou la surcharge de graisse viscérale.

5. Fructooligosaccharides (FOS) pour leur utilisation selon l'une quelconque des revendications **2** à **4,** dans laquelle la quantité efficace pour l'animal femelle lors de la gestation et/ou l'allaitement de sa progéniture est de 1 à 20 g de FOS par jour.

6. Fructooligosaccharides (FOS) pour leur utilisation selon la revendication **5,** dans laquelle la quantité efficace pour l'animal femelle lors de la gestation et/ou l'allaitement de sa progéniture est de 4 à 18 g de FOS par jour.

7. Fructooligosaccharides (FOS) pour leur utilisation selon l'une quelconque des revendications **2** à **4,** dans laquelle la quantité efficace pour la progéniture est de 0,1 à 2 g de FOS par jour.

8. Fructooligosaccharides (FOS) pour leur utilisation selon la revendication **7,** dans laquelle la quantité efficace pour la progéniture est de 0,3 à 1 g de FOS par jour.

9. Fructooligosaccharides (FOS) pour leur utilisation selon l'une quelconque des revendications **2** à **8,** dans laquelle ladite progéniture est un mammifère.

10. Fructooligosaccharides (FOS) pour leur utilisation selon la revendication **9,** dans laquelle ladite progéniture est un mammifère humain.

11. Fructooligosaccharides (FOS) pour leur utilisation selon la revendication **9,** dans laquelle ladite progéniture est un mammifère non-humain.

12. Fructooligosaccharides (FOS) pour leur utilisation selon l'une quelconque des revendications **1** à **11,** dans laquelle lesdits fructooligosaccharides sont des fructooligosaccharides à chaîne courte (scFOS).

13. Composition alimentaire ou nutraceutique pour son utilisation à un âge précoce d'un animal dans la prévention des troubles métaboliques liés à un régime alimentaire déséquilibré à l'âge adulte, comprenant des fructooligosaccharides (FOS) pour leur utilisation selon l'une quelconque des revendications **1** à **12** ;
lesquels troubles métaboliques étant sélectionnés d'un groupe comprenant ou consistant en l'hyperglycémie post-prandiale, l'hyperglycémie à jeun, l'hyperlipidémie, la surcharge pondérale, la surcharge de graisse sous-cutanée, la surcharge de graisse viscérale, le catabolisme de nutriments ralenti ou leur combinaison ; et
lesdits fructooligosaccharides étant administrés audit animal d'âge précoce entre 2 et 9 semaines après sevrage.

14. Composition alimentaire ou nutraceutique pour son utilisation selon la revendication **13,** comprenant de 0,1 à 20 g desdits FOS.

15. Composition alimentaire ou nutraceutique pour son utilisation selon la revendication **13** ou la revendication **14,** comprenant de 0,05 à 2% w/w desdits FOS, en poids par rapport à ladite composition.

## Patentansprüche

1. Fructooligosaccharide (FOS) zur Verwendung in einem frühen Alter eines Tieres zur Vorbeugung von Stoffwechselstörungen, die mit einer unausgewogenen Ernährung im Erwachsenenalter verbunden sind;
wobei die Stoffwechselstörungen aus einer Gruppe ausgewählt sind, die postprandiale Hyperglykämie, Hyperglykämie in nüchternem Zustand, Hyperlipidämie, Übergewicht, überschüssiges subkutanes Fett, überschüssiges viszerales Fett, verlangsamten Nährstoffkatabolismus oder eine Kombination davon umfasst oder daraus besteht; und
wobei die Fructooligosaccharide dem Tier im frühen Alter zwischen 2 und 9 Wochen nach dem Abstillen verabreicht werden.

2. Fructooligosaccharide (FOS) zur Verwendung nach Anspruch **1,** wobei die FOS in einer wirksamen Menge verabreicht werden an
i) ein weibliches Tier während der Trächtigkeit und/oder der Stillzeit seines Nachwuchs und an
ii) den Nachwuchs für mindestens zwei Wochen nach dem Abstillen.

3. Fructooligosaccharide (FOS) zur Verwendung nach Anspruch **1** oder Anspruch **2,** wobei die Stoffwechselstörungen aus einer Gruppe ausgewählt sind, die postprandiale Hyperglykämie, Hyperglykämie in nüchternem Zustand, Hyperlipidämie, Übergewicht, überschüssiges subkutanes Fett oder überschüssiges viszerales Fett umfasst oder daraus besteht.

4. Fructooligosaccharide (FOS) zur Verwendung nach Anspruch **3,** wobei die Stoffwechselstörungen aus einer Gruppe ausgewählt sind, die postprandiale Hyperglykämie, Hyperglykämie in nüchternem Zustand, Übergewicht, überschüssiges subkutanes Fett oder überschüssiges viszerales Fett umfasst oder daraus besteht.

5. Fructooligosaccharide (FOS) zur Verwendung nach einem der Ansprüche **2** bis **4,** wobei die wirksame Menge für das weibliche Tier während der Trächtigkeit und/oder Stillzeit seines Nachwuchs 1 bis 20 g FOS pro Tag beträgt.

6. Fructooligosaccharide (FOS) zur Verwendung nach Anspruch **5,** wobei die wirksame Menge für das weibliche Tier während der Trächtigkeit und/oder Stillzeit seines Nachwuchs 4 bis 18 g FOS pro Tag beträgt.

7. Fructooligosaccharide (FOS) zur Verwendung nach einem der Ansprüche **2** bis **4,** wobei die wirksame Menge für den Nachwuchs 0,1 bis 2 g FOS pro Tag beträgt.

8. Fructooligosaccharide (FOS) zur Verwendung nach Anspruch **7,** wobei die wirksame Menge für den Nachwuchs 0,3 bis 1 g FOS pro Tag beträgt.

9. Fructooligosaccharide (FOS) zur Verwendung nach einem der Ansprüche **2** bis **8,** wobei der Nachwuchs ein Säugetier ist.

10. Fructooligosaccharide (FOS) zur Verwendung nach Anspruch **9,** wobei der Nachwuchs ein menschliches Säugetier ist.

11. Fructooligosaccharide (FOS) zur Verwendung nach Anspruch **9,** wobei der Nachwuchs ein nicht menschliches Säugetier ist.

12. Fructooligosaccharide (FOS) zur Verwendung nach einem der Ansprüche **1** bis **11,** wobei die Fructooligosaccharide kurzkettige Fructooligosaccharide (scFOS) sind.

13. Lebensmittel- oder nutrazeutische Zusammensetzung zur Verwendung in einem frühen Alter eines Tieres zur Vorbeugung von Stoffwechselstörungen, die mit einer unausgewogenen Ernährung im Erwachsenenalter verbunden sind, umfassend Fructooligosaccharide (FOS) zur Verwendung nach einem der Ansprüche **1** bis **12;**
wobei die Stoffwechselstörungen aus einer Gruppe ausgewählt sind, die postprandiale Hyperglykämie, Hyperglykämie in nüchternem Zustand, Hyperlipidämie, Übergewicht, überschüssiges subkutanes Fett, überschüssiges viszerales Fett, verlangsamten Nährstoffkatabolismus oder eine Kombination davon umfasst oder daraus besteht; und
wobei die Fructooligosaccharide dem Tier im frühen Alter zwischen 2 und 9 Wochen nach dem Abstillen verabreicht werden.

14. Lebensmittel- oder nutrazeutische Zusammensetzung zur Verwendung nach Anspruch **13,** umfassend 0,1 bis 20 g der FOS.

15. Lebensmittel- oder nutrazeutische Zusammensetzung zur Verwendung nach Anspruch **13** oder Anspruch **14,** umfassend 0,05 bis 2 Gew.-% der FOS, bezogen auf das Gewicht der Zusammensetzung.

## Claims

1. Fructooligosaccharides (FOS) for use at an early age of an animal in the prevention of metabolic disorders linked to an unbalanced diet in adulthood;
said metabolic disorders being selected from a group comprising or consisting of postprandial hyperglycaemia, fasting hyperglycaemia, hyperlipidaemia, excess weight, excess subcutaneous fat, excess visceral fat, slowed nutrient catabolism or a combination thereof; and
said fructooligosaccharides being administered to said early-aged animal between 2 and 9 weeks after weaning.

2. Fructooligosaccharides (FOS) for the use according to claim **1,** said FOS being administered in an effective amount to
i) a female animal during gestation and/or nursing of her offspring and to
ii) said offspring for at least two weeks after weaning.

3. Fructooligosaccharides (FOS) for the use according to claim **1** or claim **2,** said metabolic disorders being selected from a group comprising or consisting of postprandial hyperglycaemia, fasting hyperglycaemia, hyperlipidaemia, excess weight, excess subcutaneous fat or excess visceral fat.

4. Fructooligosaccharides (FOS) for the use according to claim **3,** said metabolic disorders being selected from a group comprising or consisting of postprandial hyperglycaemia, fasting hyperglycaemia, excess weight, excess subcutaneous fat or excess visceral fat.

5. Fructooligosaccharides (FOS) for the use according to any one of claims **2** to **4,** wherein the effective amount for the female animal during gestation and/or lactation of her offspring is from 1 to 20 g of FOS per day.

6. Fructooligosaccharides (FOS) for the use according to claim **5,** wherein the effective amount for the female animal during gestation and/or lactation of her offspring is from 4 to 18 g of FOS per day.

7. Fructooligosaccharides (FOS) for the use according to any one of claims **2** to **4,** wherein the effective amount for the offspring is from 0.1 to 2 g of FOS per day.

8. Fructooligosaccharides (FOS) for the use according to claim **7,** wherein the effective amount for the offspring is from 0.3 to 1 g of FOS per day.

9. Fructooligosaccharides (FOS) for the use according to any one of claims **2** to **8,** wherein said offspring is a mammal.

10. Fructooligosaccharides (FOS) for the use according to claim **9,** wherein said offspring is a human mammal.

11. Fructooligosaccharides (FOS) for the use according to claim **9,** wherein said offspring is a non-human mammal.

12. Fructooligosaccharides (FOS) for the use according to any one of claims **1** to **11,** wherein said fructooligosaccharides are short-chain fructooligosaccharides (scFOS).

13. Food or nutraceutical composition for the use at an early age of an animal in the prevention of metabolic disorders linked to an unbalanced diet in adulthood, comprising fructooligosaccharides (FOS) for the use according to any one of claims **1** to **12;**
said metabolic disorders being selected from a group comprising or consisting of postprandial hyperglycaemia, fasting hyperglycaemia, hyperlipidaemia, excess weight, excess subcutaneous fat, excess visceral fat, slowed nutrient catabolism or a combination thereof; and
said fructooligosaccharides being administered to said early-aged animal between 2 and 9 weeks after weaning.

14. Food or nutraceutical composition for the use according to claim **13,** comprising from 0.1 to 20 g of said FOS.

15. Food or nutraceutical composition for the use according to claim **13** or claim **14,** comprising from 0.05 to 2% w/w of said FOS, by weight relative to said composition.
